# EUROPÄISCHE PATENTSCHRIFT

(11) **EP 1 153 397 B1**
(45) Veröffentlichungstag und Bekanntmachung des Hinweises auf die Patenterteilung: **07.09.2005**
(21) Anmeldenummer: 00904896.8
(22) Anmeldetag: 12.01.2000
(51) Int. Cl.: G21K 1/04

(54) **KOLLIMATOR ZUM BEGRENZEN EINES BÜNDELS ENERGIEREICHER STRAHLEN**
COLLIMATOR FOR LIMITING A BUNDLE OF HIGH-ENERGY RAYS
COLLIMATEUR POUR LIMITER UN FAISCEAU DE RAYONS A GRANDE ENERGIE

(30) Priorität: 12.02.1999 DE 19905823
(43) Veröffentlichungstag der Anmeldung: 14.11.2001
(73) Patentinhaber: Deutsches Krebsforschungszentrum Stiftung des öffentlichen Rechts, 69120 Heidelberg (DE)
(72) Erfinder: PASTYR, Otto, D-69181 Leimen (DE); ECHNER, Gernot, D-69257 Wiesenbach (DE); SCHLEGEL, Wolfgang, D-69118 Heidelberg (DE); GANTER, Walter, D-69190 Walldorf (DE)
(74) Vertreter: Weber, Walter
(86) Internationale Anmeldenummer: PCT/EP2000/000153
(87) Internationale Veröffentlichungsnummer: WO 2000/048203

(56) Entgegenhaltungen:
- EP-A- 0 286 858
- EP-A- 0 314 214
- EP-A- 0 387 921
- FR-A- 2 519 465

## Beschreibung

Die Erfindung betrifft einen Multileaf-Kollimator zum Begrenzen eines Bündels energiereicher Strahlen, das von einer im wesentlichen punktförmigen Strahlungsquelle ausgehend auf ein Behandlungsobjekt gerichtet ist und insbesondere der stereotaktischen Konformationsbestrahlung von Tumoren dient, wobei der Kollimator eine Vielzahl einander gegenüberliegender Blendenblätter aus strahlungsabsorbierendem Material enthält, welche durch Antriebe derart in den Strahlengang bringbar sind, daß dieser bezüglich seiner Kontur beliebig begrenzbar ist und dabei die Vorderkanten der Blendenblätter immer parallel zum Strahlengang ausgerichtet sind.

Die heute in der onkologischen Strahlentherapie eingesetzten Behandlungsgeräte sind mit Kollimatoren ausgestattet, die energiereiche Strahlen, meistens hochenergetische Gammastrahlung eines Linearbeschleunigers, derart begrenzen, daß die Strahlen exakt die Form des Behandlungsobjekts haben. Da eine derartige Bestrahlung, z. B. eines Tumors, aus verschiedenen Richtungen erfolgt, ist es möglich, eine hohe Bestrahlungsintensität des Tumors zu erreichen und trotzdem das umliegende Gewebe nur begrenzt zu belasten. Da zur Absorption der hochenergetischen Strahlung durch den Kollimator eine Dicke desselben von mehreren Zentimetern erforderlich ist, entsteht bei einer Durchlaßöffnung mit in Durchtrittsrichtung geraden Wänden ein Halbschatten. Dies resultiert daraus, daß von der im wesentlichen punktförmigen Strahlungsquelle die Strahlung auseinanderläuft, vom Kollimator die Form des Tumors in verkleinertem Maßstab erhält und danach bis zum Tumor weiter auseinanderläuft, um beim Auftreffen auf diesen exakt dessen Größe zu haben. Durch den schrägen Verlauf der Strahlung wird bei Wänden der Kollimatoröffnung, die in Durchtrittsrichtung gerade sind, ein Teil der Strahlung nicht durch die volle Materialstärke abgeschirmt. Dies führt dazu, daß um den Tumor herum ein Rand gesunden Gewebes mit erheblicher Strahlung belastet oder das Tumorgewebe mit zu geringer Dosis bestrahlt wird. Dadurch kommt es zu einer Schädigung, die vermieden werden sollte. Aus diesem Grund wurden verschiedene Möglichkeiten ersonnen, Kollimatoren zu entwickeln, die diesen Halbschatten reduzieren oder vermeiden.
Ein Vorschlag zur Vermeidung des Halbschattens, der durch eine Vielzahl von Schriften gemacht wurde, besteht darin, dun Blendenblättern (Leafs) eines Kollimators (Multileaf-Kollimator) eine derartige Form unregelmäßiger Trapeze zu geben, daß deren Seitenflächen sowie die Seitenflächen der äußeren Begrenzungen der Kollimatoröffung den Winkel des Strahlengangs aufweisen. Schwieriger ist es jedoch, eine entsprechende Ausrichtung der Vorderkanten der Kollimatorblätter zu erreichen. Zur Lösung dieses Problems wurden eine Vielzahl von Vorschlägen gemacht, die jedoch alle nicht befriedigend sind.

Ein Vorschlag, der beispielsweise von den Schriften EP 0 259 989 B1, EP 0 556 874 B1, EP 0 562 644 B1, US 5 166 531 und DE 33 11 870 C2 gemacht wurde, besteht darin, den Vorderkanten der Kollimatorblätter in einer derart gerundeten Form auszubilden, daß die äußeren Strahlen des Strahlenbündels diese Vorderkanten tangential berühren. Durch diese Lösung läßt sich der Halbschatten zwar abschwächen, jedoch nicht verhindern. Dasselbe gilt für einen weiteren Vorschlag der EP 0 259 989 B1, EP 0 556 874 B1 und EP 0 562 644 B1, bei dem die Strahlung nacheinander zwei Kollimatoröffnungen passieren muß. Eine derartige Stufung der Vorderkanten der Kollimatorblätter wurde durch die DE 195 04 054 A1 noch verfeinert, indem jedes Kollimatorblatt aus einer Vielzahl von Stäben besteht, welche gegenseitig verschiebbar übereinander gelagert sind. Dieser Kollimator ist jedoch durch die Vielzahl von Teilen kompliziert und weist aufgrund der Aneinandergrenzungen vieler Blendenblattelemente und den dabei unvermeidbaren Toleranzen eine erhöhte Leckstrablung auf. Außerdem sind keine Antriebe vorgesehen, so daß Anpassungen manuell vorgenommen werden müssen und eine ständige computergesteuerte Anpassung der Kollimatorüffnung nicht möglich ist.

Schließlich wurde von den Schriften DE 33 11 870 C2, US 3 151 245, US 4 987 309, US 5 144 647, EP 0 193 509, EP 0 245 768 B1, der im wesentlichen identischen EP 0 387 921 A und EP 0 314 214 B1 vorgeschlagen, die Kollimatorblätter derart auf bogenförmigen Bahnen zu bewegen, daß die Vorderkanten der Blendenblätter immer parallel zum Strahlengang ausgerichtet sind. Um dies zu erreichen, sind jedoch komplizierte Führungen der Blendenblätter erforderlich. Die Unterbringung solcher komplizierter Führungen setzt jedoch dem Bestreben, die Kollimatorblätter möglichst dünn zu machen, eine Grenze. Dünne Kollimatorblätter sind jedoch erforderlich, um die Gestalt des Tumors exakt nachzubilden, denn Grob-abstufungen führen dazu, daß gesundes Gewebe milbestrahlt und zerstört oder schwer geschädigt wird. Außerdem kommt es, wenn Kollimatorblätter in der Form von unregelmäßigen Trapezen ausgebildet und auf bogenförmigen Bahnen geführt werden, aus geometrischen Gründen zu Verkeilungen. Um diese zu vermeiden, wurde von der DE 37 11 245 A1 vorgeschlagen, daß sich die Kollimatorblätter zu ihrem vorderen, dem Strahlengang zugewandten Ende etwas verjüngen. Wird ein solcher Kollimator weit geöffnet, entstehen Spalten, die erhöhte Leckstrahlung zur Folge haben. Schließlich wurde die Lösung des genannten Problems von der EP 0 314 214 B1 und der US 4 987 309 darin gesehen, übereinander die trapezförmigen Kollimatorblätter und die auf bogenförmigen Bahnen verschiebbaren Kollimatorblätter anzuordnen, damit das Strahlenbündel beide Kollimatoröffnungen passieren muß. Jede der zwei Blenden hat einen Halbschatten, der zwar jeweils durch die andere Blende vermindert wird, jedoch nur beseitigbar ist, wenn man mit den beiden Blenden einen doppelten Aufwand betreibt, also nahezu die volle Materialstärke zweimal anordnet. Auch der Antriebs- und Steuerungsaufwand verdoppelt sich.

Weiterhin sind aus der FR-A-2 519 465 und der EP-A-0 286 858 Kollimatoren bekannt, die aus zwei um 90" versetzte Paarc von Abschirmungsblöcken bestehen. Diese Abschirmungsblöcke weisen zur Vermeidung eines Halbschattens hintere und vordere Teilstücke auf, wobei letztere parallel zum Strahlengang ausgerichtet werden können. Die vorderen Blöcke weisen seitlich herausragende Lagerzapfen zur Anlenkung an mit den hinteren Blöcken verbundenen Halteeinrichtungen auf, an die auch die Antriebsmittel für die Stellbewegung angreifen. Mit derartigen Kollimatoren ist jedoch nur eine viereckige Formung eines Strahls möglich. Die Erzeugung einer Kontur in der Form eines Behandlungsobjekts in einem lebenden Organismus, wie beispielsweise eines Tumors, ist nicht möglich, dazu bedarf es eines konturbildenden Multileafkollimators der eingangs genannten Art. Eine Übertragung der vorgeschlagenen technischen Lösung auf einen Multileafkollimator steht entgegen, daß eine seitliche Lagerung vorderer Teilstücke der genannten Art bei Blendenblättern eines Multileafkollimators nicht an einer Stelle möglich ist, an der sich das nächste Blendenblatt (Leaf) befinden muß, um die eingangs genannte Kontur zu bilden. Für Multileafkollimatoren ist es ein ganz wesentliches Erfordernis, daß es zwischen den die Kontur bildenden Blendenblättern zu keiner Unterbrechung der Abschirmung kommt, da derartige Leckstrahlen gesundes Gewebe zerstören würden.

Der Erfindung liegt daher die Aufgabe zugrunde, die genannten Probleme zu lösen und mit möglichst geringem Aufwand einen Multileaf-Kollimator zu schaffen, bei dem kein Halbschatten auftritt.

Die Aufgabe wird erfindungsgemäß dadurch gelöst, daß die Blendenblätter aus einem geradlinig verschiebbar gelagerten hinteren Teilstück und einem an diesem angelenkten vorderen Teilstück bestehen, wobei das vordere Teilstück eines jeden Blendenblattes durch Antriebsmittel eine derartige, der jeweiligen Position des jeweiligen hinteren Teilstücks zugeordnete Stellbewegung erfährt, daß die Vorderkanten immer parallel zum Strahlengang ausgerichtet sind und daß die Anlenkung des vorderen Teilstücks an das hintere Teilstück derart erfolgt, daß dadurch keine nennenswerte Unterbrechung des Volumens des strahlungsabsorbierenden Materials eintritt.

Durch die Erfindung entfällt die komplizierte bogenförmige Verschiebung der Kollimatorblätter. Dadurch wird sowohl die Mechanik einfacher, als auch die Leckstrahlung vermindert, da die geradlinige Verschiebung engere Toleranzen zuläßt. Die Stellbewegung des vorderen Teilstücks läßt sich von der Mechanik und der Antriebstechnik wesentlich besser und einfacher realisieren als die bogenförmige Verschiebung. Es wird ganzflächig die volle Materialstärke zur Abschirmung eingesetzt, wodurch weder ein Halbschatten auftritt, auch kein vermindeter Halbschatten, noch ein erhöhter Aufwand durch eine zusätzliche Abschirmung wie beim oben genannten Stand der Technik erforderlich ist. Die vorgeschlagene technische Lösung zeigt ihre Überlegenheit gegenüber der bisherigen Lösungsvorschlägen insbesondere, wenn die Blendenblätter in Form von Trapezen ausgebildet sind, um auch den Halbschatten durch die Seitenflächen der Blendenblätter zu vermeiden. Die geradlinige Führung kann auch bei der trapezförmigen Gestalt der Blendenblätter nicht zum Verkeilen führen. Dadurch sind engere Toleranzen möglich, und die Leckstrahlung wird gegenüber Blendenblättern, die bogenförmig geführt werden, vermindert. Lediglich die vorderen Teilstücke brauchen eine etwas erhöhte Toleranz, um die Stellbewegung nicht durch die Trapezform zu behindern. Diese Toleranz ist jedoch im Verhältnis zu der bogenförmigen Führung sehr gering. Selbstverständlich ist die Erfindung nicht auf trapezförmige Blendenblätter festgelegt. Dies hängt von der Größe des Kollimators und dem Winkel des Strahlengangs ab.

Die Erfindung sicht vor, daß die Anlenkung des vorderen Teilstücks an das hintere Teilstück derart erfolgt, daß dadurch keine nennenswerte Unterbrechung des Volumens des strahlenabsorbierenden Materials eintritt. Dies sollte bei der konkreten Ausführung der Anlenkung beachtet werden, wobei es dafür mehrere Möglichkeiten gibt, die noch erläutert werden.

Es ist möglich, für die Verschiebung der hinteren Teilstücke der Blendenblätter und für die Stellbewegung der vorderen Teilstücke separate Antriebe vorzusehen, wobei diese rechnergesteuert aufeinander abgestimmt werden. Vorzugsweise werden die Antriebsmittel jedoch derart ausgebildet, daß eine mechanische Zwangskopplung zu jeder Position des hinteren Teilstücks die zugehörige Ausrichtung des vorderen Teilstücks und damit der Vorderkanten garantiert. Auf diese Weise kann eine Fehlausrichtung der Vorderkanten durch Fehler im Programm oder in den Antrichsmitreln nicht auftreten. Die Zuverlässigkeit ist wesentlich erhöht, was für die Patienten und die Anwender besonders wichtig ist. Weitere Vorteile dieser Ausführungsform bestehen darin, daß für jedes Blendenblatt nur ein Antrieb erforderlich ist. Der Rechneraufwand ist entsprechend geringer, wodurch das Rechenergebnis schneller bereit steht und dadurch der Kollimator wesentlich schneller auf eine andere Kontur umgestellt werden kann.

Für die oben genannte Anlenkung des vorderen Teilstücks an das hintere Teilstück gibt es eine ganze Reihe von Möglichkeiten, beispielsweise kann das Ende des hinteren Teilstücks halbrund ausgestaltet sein und darauf ein korrespondierendes vorderes Teilstück in der halbrunden Ausnehmung angeordnet werden. Es ist auch möglich, segrnentförmige vordere Teilstücke mit entsprechenden Ausnehmungen der hinteren Teilstücke zu kombinieren. Vorzugsweise wird jedoch vorgesehen, daß die vorderen Teilstücke im wesentlichen halbkreisförmige Körper sind, die in korrespondierende Ausnehmungen am vorderen Ende der hinteren Teilstücke unverlierbar gelagert sind, wobei die Stellbewegung eine Schwenkung um die im Mittelpunkt der Kreislinie liegende gedachte Drehachse ist. Es bestehen verschiedene Möglichkeiten der unverlierbaren Lagerung ohne nennenswerte Unterbrechung des Volumens des strahlungsabsorbierenden Materials, beispielsweise Schwalbenschwanzführungen, Führungen in Nuten mit in Langlöchern geführten Haltezapfen, usw. Vorzugsweise entspricht die Höhe des hinteren Teilstücks im wesentlichen dem Durchmesser des halbkreisförmigen Körpers, wobei die vorderen Enden des hinteren Teilstücks derart zurückversetzt sind, daß jede erforderliche Schrägstellung der Vorderkanten der Blendenblätter möglich ist. Diese Ausführungsfonn hat den Vorteil, daß auch das angelenkte vordere Teilstück in jeder möglichen Position dieselbe Höhe wie das zugehörige hintere Teilstück aufweist. Dies ist für die konkrete Ausgestaltung der Lagerung und Führung der Blendenblätter von Vorteil.

Vorteilhafterweise weisen die Querschnitte der Blendenblätter eine derartige asymmetrische Trapezformen auf, daß deren Seitenflächen in etwa parallel zum Strahlengang verlaufen, wobei die Innenflächen der an die äußeren Blendenblätter anschließenden seitlichen Begrenzungen derart schräg verlaufen, daß sie an diese äußeren Blendenblätter lückenfrei anschließen. Auf diese Weise wird ein Halbschatten vennieden, da alle Begrenzungen der Kollimatoröffnung in etwa dem Strahlengang entsprechen. Daß die Erfindung gerade für eine derartige Ausgestaltung des Kollimators von großem Vorteil ist, wurde bereits oben erwähnt. Vorzugsweise weisen dabei die vorderen Teilstücke soviel seitliches Spiel auf, daß deren Stellbewegung trotz der Trapezform möglich ist.

Eine Ausgestaltung sieht vor, daß die Blendenblätter über die Mittellinie der möglichen Kollimatoröffnung hinaus verschiebbar sind. Dadurch ist es möglich, alle unregelmäßigen Konturen eines Tumors nachzubilden, beispielsweise auch Formen die einer U-Form ähnlich sind, und deshalb zur Nachbildung einer solchen Form die Blendenblätter über die Mittellinie der Kollimatoröffnung gehen müssen. Es kann durch diese Ausführungsform auch eine Intensitätsmodulierung des Strahls durch zeitweise Abdeckung bestimmter Bereiche leichter vorgenommen werden. Ein weiterer Vorteil besteht darin, daß die Blendenblätter asymmetrisch geschlossen werden können, beispielsweise in der Art eines Reißverschlusses. Durch letzteres wird die Leckstrahlung im Schließbereich wesentlich geringer als bei einer Schließung aller Blendenblätter in der Mitte. Selbstverständlich muß, um eine solche Verschiebung über die Mittellinie zu erreichen, eine entsprechende Länge und ein entsprechend großer Fahrweg der Blendenblätter vorgesehen sein.

Für den Antrieb der Blendenblätter kann wie bei der eingangs erwähnten EP 0 245 768 B1 und der im wesentlichen identischen EP 0 387 921 A1 vorgesehen sein, daß ein Antriebsorgan nacheinander mehrere Blendenblätter verstellt. Vorzugsweise wird jedoch vorgesehen, daß jedes Blendenblatt einen steuerbaren Einzelantrieb aufweist. Dadurch ist es möglich, rechnergesteuert schnelle Formänderungen berbeizuführen, was bei dynamischer Bestrahlung eines Tumors besonders wichtig ist. Dadurch ist eine relativ schnell wechselnde Bestrahlung von verschiedenen Seiten möglich, auch wenn das Bestrahlungsobjekt irregulär geformt ist und ein schneller Wechsel der Kontur erfolgen muß. Dadurch ist es möglich, das umliegende Gewebe maximal zu schonen. Die Einzelantriebe sind auch zweckmäßig, wenn Blendenblätter während der Bestrahlung zeitweise in die Kollimatoröffnung gefahren werden sollen, um die Intensität der Bestrahlung für gewisse Teilbereiche abzuschwächen. Zur Erhöhung der Sicherheit und zur Begrenzung der Anzahl der Antriebe ist es zweckmäßig, wenn diese Einzelantriebe mit der bereits oben erwähnten Zwangskopplung kombiniert werden.

Bevorzugterweise erfolgt die Steuerung des Kollimators beim Betrieb desselben über einen Rechner, der die Kontur und Lage der Kollimatoröffnung dem Bestrahlungsobjekt in der jeweiligen Bestrahlungsrichtung anpaßt, wobei der Rechner die Daten von einer Vorrichtung zur Erfassung der Gestalt des Bestrahlungsobjekts erhält und eine Kontrolleinrichtung das Ergebnis der Stellbewegung überprüft. Dabei können die Blendenblätter in einem zweck mäßigerweise verschiebbaren Kollimatorblock gelagert sein, der zur Positionierung der Kollimatoröffnung relativ zum Bestrahlungsobjekt und zur Strahlungsquelle dient. Der Kollimatorblock kann auch entlang einer Mittellinie geteilt sein, wodurch es möglich ist, diese Hälften getrennt zu verschiehen. Werden beide Hälften auseinandergezogen, so läßt sich dadurch die Kollimatoröffnung zusätzlich vergrößern. Weiterhin kann der Kollimatorblock an einer Gantry befestigt sein, wobei eine derartige Relativbewegung zwischen Kollimatorblock und Patienten möglich ist, daß der Patient unter Anpassung der Kollimatoröffnung an die Gestalt des Bestrahlungsobjekts von allen Seiten bestrahlbar ist. Auf diese Weise läßt sich der Kollimator zum Umkreisen eines zu bestrahlenden Tumors einsetzen, wobei diese Umkreisung nicht nur eine Kreisbewegung zu sein braucht, sondern auch räumlich ausgeführt werden kann. Eine derartige Bestrahlungsmethode ist zwar bekannt, in Kombination mit der Erfindung läßt sie sich jedoch wesentlich besser vornehmen, da der erfindungsgemäße Kollimator leichter gebaut und angetrieben werden kann und auch der Rechneraufwand wesentlich vermindert ist. Vor allem erfordert diese Methode eine hohe Sicherheit gegen Fehlfunktionen, welche durch die Zwangskopplung der beiden Antriebe erzielt ist.

Diese Zwangskopplung des Antriebs der hinteren Teilstücke der Blendenblätter mit dem Stellantrieb für die vorderen Teilstücke kann über ein Getriebe erfolgen. Um mehr Bauraum für die Getriebe zur Verfügung zu haben, kann dabei vorgesehen sein, daß die Getriebe, gegebenenfalls auch die Antriebe, für die Blendenblätter wechselweise bei einem Blendenblatt oberhalb und heim angrenzenden Blendenblatt unterhalb angeordnet sind. Dies ist besonders wichtig, wenn die Blendenblätter sehr schmal sein sollen, was für eine exakte Nachbildung der Gestalt des Tumors erforderlich ist. Eine erste Ausgestaltung der Antriebe sieht vor, daß der Stellantrieb für die vorderen Teilstücke derart ausgestaltet ist, daß er letztere sowohl bei einer individuellen Verstellung der Blendenblätter als auch bei einer Verstellung aller oder eines Teils der Blendenblätter gegenüber der Strahlungsquelle ausrichtet. Dadurch kann auch der Kollimatorblock insgesamt verfahren werden oder es ist möglich, durch Verstellung der Kollimatorblockhälften diese auseinanderzufahren und dadurch eine größere Kollimatoröffnung zu erreichen. Dadurch können mit einem relativ kleinen Kollimator auch größere Bestrahlungsobjekte behandelt werden, ohne daß auf die erfindungsgemäße Ausrichtung der Vorderkanten der Blendenblätter verzichtet werden muß.

Für den Antrieb kann dem hinteren Teilstück eine Blendenzahnstange zugeordnet sein, in die ein Antriebszahnrad eingreift, wobei die dem hinteren Teilstück zugeordnete Blendenzahnstange auch als Verzahnung einer Längskante des hinteren Teilstücks der Blendenblätter ausgeführt sein kann.

Um die Blendenblätter gut zu führen, kann vorgesehen sein, daß im Bereich der Verzahnung der Längskante eines hinteren Teilstücks das an dieses angrenzende hintere Teilstück derart in der Höhe versetzt im Kollimatorblock gelagert ist, daß oberhalb der Verzahnung seitlich ein mit dem Kollimatorblock in Verbindung stehendes Führungselement in eine Führungsnut des hinteren Teilstücks eingreifen kann. Auf diese Weise wird eine sichere Führung unmittelbar in der Nähe des Eingriffsbereiches des Zahnrads erzielt, was einer exakten, spielfreien Verschiebung der Blendenblätter dient. Selbstverständlich können weitere Führungen vorgesehen sein, so ist es zum Beispiel möglich, daß die der Verzahnung gegenüberliegende Kante des hinteren Teilstücks ebenfalls geführt wird. Besonders zweckmäßig ist dabei ein in einer Nut der Längskante des hinteren Teilstücks geführtes Führungselement, da dadurch das Blendenblatt auch dann sicher in seiner Position gehalten wird, wenn das angrenzende Blendenblatt eine wesentlich andere Position einnimmt und sich daher nicht unmittelbar anschließt.

Als Antriebsmittel für die Stellbewegung des vorderen Teilstücks kann vorgesehen sein, daß an dieses außerhalb von dessen Drehachse eine Vorderkantenzahnstange angelenkt ist, in die ein Zahnrad eingreift und einen gegenüber dem hinteren Teilstück abweichenden Stellweg erzielt. Der abweichende Stellweg ist derart, daß die entsprechende Ausrichtung der Vorderkante erfolgt. Dies kann zum Beispiel durch separate Antriebe erfolgen. Bevorzugt wird jedoch die mechanische Zwangskopplung,

Eine einfach aufgebaute und sicher funktionierende Ausführungsform sieht dabei vor, daß die Blendenzahnstange und die Vorderkantenzahnstange an einer Längskante des hinteren Teilstücks angeordnet sind und zur Erzielung des abweichenden Stellwegs eine unterschiedliche Teilung aufweisen, wobei ein Zahnrad in beide Zahnstangen eingreift und dabei der Teilungsunterschied im Toleranzbereich der Verzahnung liegt. Für ein unterhalb einer Blende angeordnetes Getriebe ist dabei vorgesehen, daß die Teilung der Vorderkantenzahnstange größer ist als die Teilung der Blendenzahnstange. Für ein oberhalb einer Blende angeordnetes Getriebe muß die Teilung der Vorderkantenzahnstange kleiner sein als die Teilung der Blendenzahnstange. Diese Ausführungsform ist selbstverständlich nur beispielhaft, weitere Möglichkeiten sind denkbar, zum Beispiel Spindelantriebe mit unterschiedlichen Steigungen.

Die Antriebszahnräder oder die weiteren Zahnräder, die in beide Zahnstangen eingreifen, können im Kollimatorblock gelagert sein, die weiteren Zahnräder auch im Grundgestell. Dabei ist es möglich, daß ein Zahnrad beide Funktionen übernimmt oder daß zwei separate Zahnräder vorgesehen werden.

Eine Weiterbildung sieht vor, daß das Antriebszahnrad, welches in eine Antriebszahnstange eingreift, in einem verschiebbaren Kollimatorblock oder zwei verschiebbaren Kollimatorblockhälften und ein weiteres Zahnrad, das in die Blendenzahnstange und die Vorderkantenzahnstange eingreift, auf einem Grundgestell gelagert sind. Auf dem Grundgestell ist der Kollimatorblock insgesamt oder die Kollimatorblockhälften - für je einen Teil der Blendenblätter - verschiebbar. Dabei kann das Grundgestell zwischen Kollimatorblock und Gantry angeordnet sein, oder es kann sich bei dem Grundgestell um die Gantry selbst handeln. Bei der Antriebszahnstange kann es sich um eine separate Zahnstange handeln oder um eine Weiterführung der Blendenzahnstange gegenüber einer kürzer ausgebildeten Vorderkantenzahnstange. Der Vorteil dieser Ausführungsform besteht darin, daß auch beim Verschieben des Kollimatorblocks oder der Kollimatorhälften eine einmal justierte Einstellung der Vorderkanten der Blendenblätter zur Strahlungsquelle erhalten bleibt. Das heißt, daß auch bei einer Verstellung des Kollimatorblocks oder der Kollimatorblockhälften die Vorderkanten immer parallel zur Strahlung ausgerichtet sind. Dies wird durch das weitere Zahnrad bewirkt, das in beide Zahnstangen eingreift und dadurch die Zuordnung im gesamten Laufbereich beider Zahnstangen gewährleistet. Auf diese Weise wird der mögliche Stellbereich und die erzielbare Kollimatoröffnung wesentlich vergrößert.

Selbstverständlich sind noch viele Arten von Steuerungen und von mechanischen Zwangskopplungen möglich. So kann ein Antrieb für die hinteren Teilstücke mit Kulissensteuerungen für die Stellbewegung der vorderen Teilstücke verbunden sein. Derartige Kulissensteuerungen können ebenfalls verschieden ausgestaltet werden. So kann die Kulissenführung mit der Lagerung des Antriebszahnrades in fester Verbindung und das Gleitstück der Kulissensteuerung mit dem vorderen Teilstück in Wirkverbindung stehen. Es ist jedoch auch möglich, daß die Kulissenführungen mit einem Grundgestell in fester Verbindung stehen und verschiebbare Kollimatorblockhälften - für je einen Teil der Blendenblätter - mit den Lagerungen der Antriebszahnräder in fester Verbindung stehen. Eine konkrete Ausgestaltung einer Kulissenführung sieht vor, daß das Gleitstück an einem Seilzug befestigt ist, der zum vorderen Teilstück geführt und mit einem Ende oberhalb und dem anderen Ende unterhalb der gedachten Drehachse des vorderen Teilstücks befestigt ist. Eine weitere Ausführungsmöglichkeit besteht darin, daß das Gleitstück an einem hinteren Ende eines doppelarmigen Hebels befestigt ist, wobei der Hebel mit seiner Drehachse auf dem hinteren Teilstück gelagert und mit seinem vorderen Ende im hinteren Bereich des vorderen Teilstücks zur Ausführung der Stellbewegung angreift.

Zweckmäßigerweise ist an mindestens einer, vorzugsweise an beiden Längskanten jedes hinteren Teilstücks eine Führung vorgesehen. Diese kann beispielsweise derart ausgestaltet sein, daß an der Längskante eine Nut eingearbeitet ist, in der ein Führungselement des Kollimatorblocks gleitet. Weitere Möglichkeiten von Führungen sind denkbar, wobei gewährleistet sein muß, daß ein Blendenblatt auch dann sicher geführt ist, wenn das angrenzende Blendenblatt derart stark verschoben ist, daß das Blendenblatt an dieser Stelle freiliegt.

Weiterhin kann vorgesehen sein, daß die Blendenblätter als Kompensatormittel zur Erzeugung unterschiedlicher Strahlungsintensität dienen, indem sie während der Bestrahlung zeitweise in die Kollimatoröffnung einfahrbar sind. Auf diese Weise können separate Kompensatormittel eingespart werden, welche selbstverständlich ebenfalls mit dem erfindungsgemäßen Kollimator kombinierbar sind.

Die der Erfindung zugrundeliegende Problematik und Ausführungsbeispiele der Erfindung werden nachfolgend anhand von Prinzipienskizzen erläutert. Es zeigen
- **Fig. 1**: den prinzipiellen Aufbau eines Bestrahlungsgeräts in dem der erfindungsgemäße Kollimator beispielsweise Ånwendung finden kann,
- **Fig. 2**: eine Kollimatoröffnung eins Multileaf-Kollimators,
- **Fig. 3a**: das Prinzip der Entstehung eines Halbschattens bei Kollimatoren nach dem Stand der Technik,
- **Fig. 3b**: das Prinzip der Vermeidung eines Halbschattens beim erfindungsgemäßen Kollimator,
- **Fig. 3c**: partielle Halbschattenvermeidung durch eine trapezförmige Ausgestaltung der Blendenblätter,
- **Fig. 4**: das Prinzip eines erfindungsgemäßen Ausführungsbeispiels,
- **Fig. 5a und 5b**: Zahnteilungen dieses Ausführungsbeispiels,
- **Fig. 6**: eine Anlenkung eines vorderen Teilstücks eines Blendenblattes,
- **Fig. 7**: eine Anordnung von Blendenblättern,
- **Fig. 8**: eine Lagerung von Blendenblättern,
- **Fig. 9a und 9b**: ein zweites Ausführungsbeispiel,
- **Fig. 10a und 10b**: ein drittes Ausführungsbeispiel
- **Fig. 11**: ein viertes Ausführungsbeispiel und
- **Fig. 12**: eine weitere Ausgestaltungsform des zweiten Ausführungs beispiels.

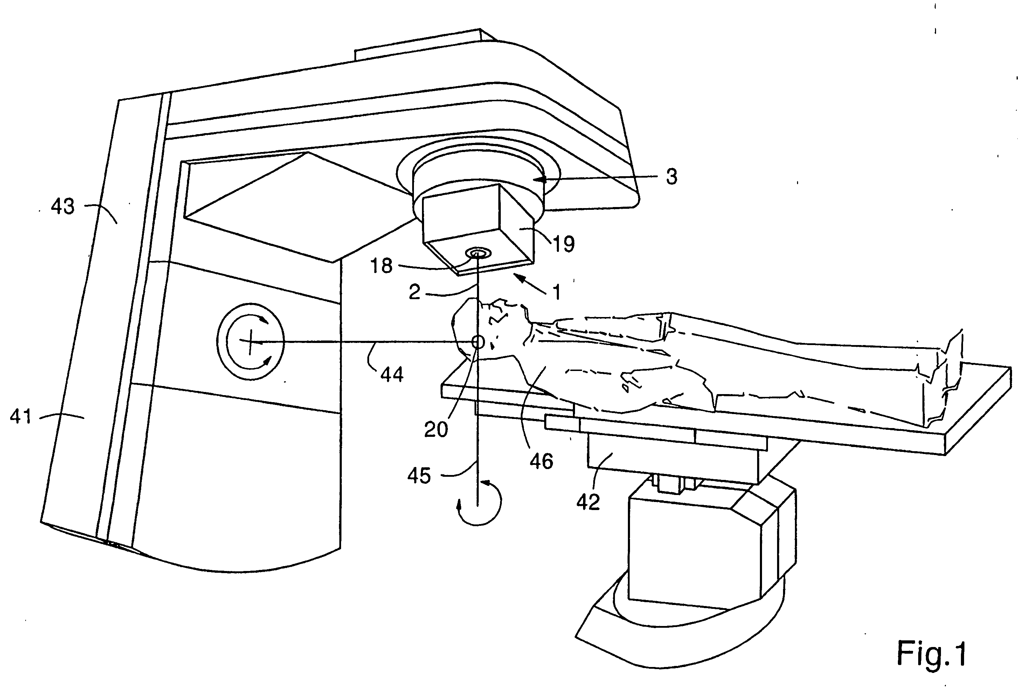

**Fig. 1** zeigt den prinzipiellen Aufbau eines Bestrahlungsgeräts, in dem der erfindungsgemäße Kollimator 1 beispielsweise Anwendung finden kann. Der Kollimator 1 befindet sich an einem Kollimatorblock 19, welcher in einer Gantry 41 gelagert ist. Die Gantry 41 enthält die Strahlungsquelle 3, wobei die Strahlung beispielsweise durch einen Linearbeschleuniger 43 erzeugt wird. Die Gantry 41 ist um eine horizontale Rotationsachse 44 drehbar, wobei die Strahlen 2 auf ein Bestrahlungsobjekt 20, beispielsweise einen Tumor, gerichtet sind. Das Bestrahlungsobjekt 20 befindet sich im Isozentrum der Strahlen 2, wobei Strahlungsquelle 3 und Kollimator 1 durch die Rotation der Gantry 41 den Patienten 46 umkreisen. Gleichzeitig kann eine Rotation des Behandlungstischs 42 um eine Rotationsachse 45 stattfinden, um eine weitere Änderung der Einstrahlung der Strahlen 2 auf das Behandlungsobjekt 20 des Patienten 46 vorzunchmen. Selbstverständlich sind noch weitere Stellbewegungen denkbar. Der Sinn besteht darin, daß durch die unterschiedlichen Bestrahlungsrichtungen das Behandlungsobjekt 20 eine maximale Bestrahlung erfährt, jedoch das umliegende Gewebe maximal geschont wird, da es immer nur kurzzeitig den Strahlen 2 ausgesetzt ist. Außerdem ist es oft erforderlich, daß bestimmte Bereiche des Körpers, wie beispielsweise das Rückenmark oder Organe, von der Bestrahlung möglichst völlig verschont und daher bei der Wahl der Bestrahlungsrichtungen ausgespart werden. Die Strahlen 2 werden außerdem von der Kollimatoröffnung 18 derart geformt, daß sie beim Bestrahlungsobjekt 20 in dessen Form eintreffen, wodurch das umliegende Gewebe geschont wird. Das Profil des Tumors wird beispielsweise durch eine Computer-Tomographie-Aufnahme erfaßt. Diese Daten werden derart umgesetzt, daß der Kollimator 1 eine dieser Form entsprechende Kollimatoröffnung 18 ausbildet und gegebenenfalls auch innerhalb des Bestrahlungsobjektes 20 mit verschiedenen Intensitäten bestrahlt wird. Form und Intensität werden für jede Bestrahlungsrichtung errechnet und eingestellt.

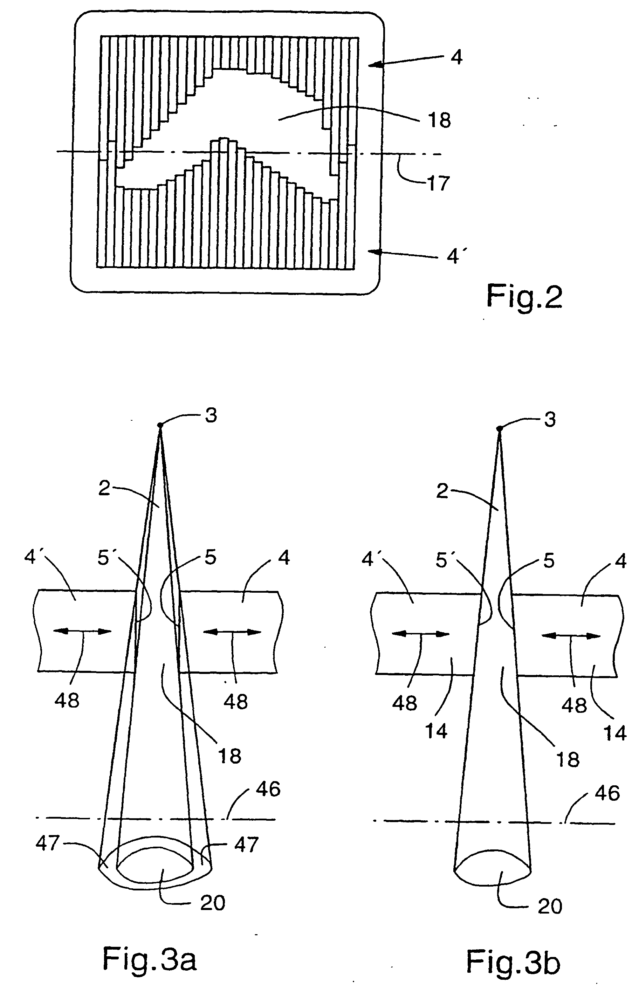

**Fig. 2** zeigt eine Kollimatoröffnung 18 eines Multileaf-Kollimators. Die Erfindung betrifft einen derartigen Kollimator 1 mit den bereits oben erwähnten und in den weiteren Figuren dargestellten und erläuterten Verbesserungen. Das Wesen der Erfindung besteht darin, daß die Blendenblätter 4 und 4' eine der Gestalt des Behandlungsobjekts 20 entsprechende Kollimatoröffnung 18 erzeugen können, ohne daß ein Halbschatten 47 auftritt. Dies wird weiter unten näher erläutert. Anhand der Figur 2 ist das Prinzip eines als Multileaf-Kollimator ausgebildeten Kollimators 1 erkennbar, der mit Hilfe von Blendenblättern 4, 4' die Gestalt eines Tumors durch die Blendenöffnung 18 nachbildet. Zweckmäßige Ausgestaltungen der Erfindung sehen dabei vor, daß die Blendenblätter 4 und 4' über die Mittellinie 17 der möglichen maximalen Kollimatoröffnung 18 geschoben werden können. Dies ist beispielsweise bei einer Form des Bestrahlungsobjektes 20 notwendig, das eine U-Form oder ähnliche Formen aufweist, die nur dann nachgebildet werden können, wenn die Blendenblätter 4 und 4' über die Mittellinie 17 gehen. Außerdem läßt sich dadurch ein Schließen der Blendenblätter 4 und 4' erzielen, wie dies am linken und rechten Rand dargestellt ist. Dabei stoßen die Blendenblätter nicht in der Mittellinie 17 aufeinander, sondern sind reißverschlußartig verzahnt, damit die Leckstrahlung in diesem Bereich vermindert ist.

**Fig. 3a** zeigt das Prinzip der Entstehung eines Halbschattens 47 bei Kollimatoren nach einem Teil des Standes der Technik. Dort weisen die Blendenblätter 4 und 4' gerade Vorderkanten 5 und 5' auf. Fallen von einer im wesentlichen punktförmigen Strahlungsquelle 3 kommende Strahlen 2 durch die Kollimatoröffnung 18 hindurch, so muß ein Teil dieser Strahlen 2 durch die gesamte Materialstärke hindurch und ein anderer Teil der Strahlen berührt das Material nicht. Im Zwischenbereich treten die Strahlen nur durch einen Teil des Materials der Blendenblätter 4, 4' hindurch und werden teilweise absorbiert, was mit Halbschatten 47 bezeichnet wird. Dieser Halbschatten 47 wird um so größer, je mehr die Blendenblätter 4 und 4' durch die Stellbewegung 48 auscinanderbewegt werden. Auf Grund dieses Halbschattens 47 wird außer dem Bestrahlungsobjekt 20 auch ein Umfeld dieses Bestrahlungsobjektes 20 mit verminderten Strahlen 2 bestrahlt. Dadurch kommt es zu Schädigungen des umliegenden Gewebes des Patienten 46. die vermieden werden sollen. Auch bei gerundeten Vorderkanten 5, 5' oder bei in Stufenform übereinander angeordneten Blendenblättern 4, 4' kann eine solche Halbschattenbildung zwar reduziert, jedoch nicht beseitigt werden.

**Fig. 3b** zeigt das Prinzip der Vermeidung eines Halbschattens 47 beim erfindungsgemäßen Kollimator 1. Durch die Erfindung sind die Blendenblätter 4 und 4', obwohl sie geradlinig verschiebbar sind, derart ausgebildet, daß ihre Vorderkanten 5 und 5' immer parallel zu den Strahlen 2 ausgerichtet sind. Auf diese Weise ist gewährleistet, daß ein Strahl 2 entweder voll durch die Kollimatoröffnung 18 hindurchgeht und dann auf das Bestrahlungsobjekt 20 trifft oder von der gesamten Materialstärke der Blendenblätter 4 und 4' absorbiert wird. Da die Ausrichtung der Vorderkanten 5 und 5' entsprechend der Stellbewegung 48 der Blendenblätter 4 und 4' erfolgt, wird diese Verhinderung eines Halbschattens 47 bei allen Öffnungsweiten der Kollimatoröffnung 18 gewährleistet.

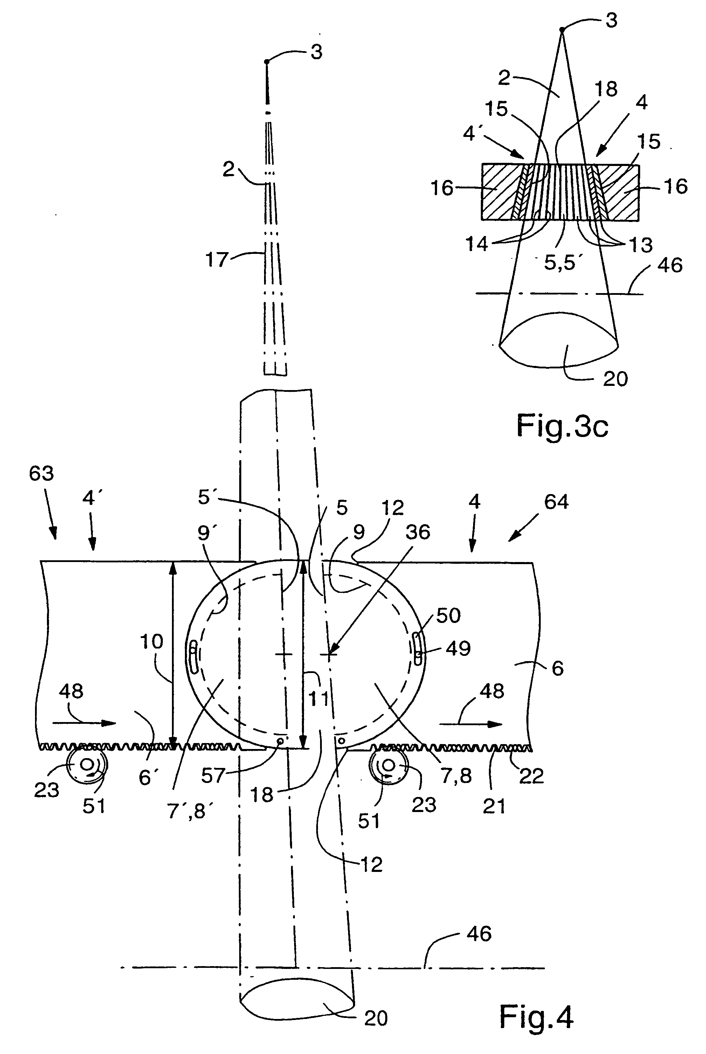

**Fig. 3c** zeigt eine partielle Halbschattenvermeidung durch eine Ausgestaltung der Blendenblätter 4 und 4' in asymmetrischer Trapezform 13. Die Blickrichtung auf den Kollimator 1 ist gegenüber den Darstellungen der Fig. 3a und 3b um 90° gedreht und auf die Vorderkanten 5, 5' gerichtet. Mit der dargestellten Ausbildung wird vermieden, daß die Seitenflächen 14 der Blendenblätter 4, 4' und die seitlichen Begrenzungen 16 einen Halbschatten 47 erzeugen. Dabei weisen die Blendenblätter 4 und 4' eine derartige asymmetrische Trapezform 13 auf, daß bei jedem Blendenblatt 4, 4' die beiden Seitenflächen 14 parallel zu den Strahlen 2 verlaufen. Auch die Innenflächen 15 der seitlichen Begrenzungen 16 weisen eine entsprechende Ausrichtung auf, wobei sie dadurch lückenlos an die Seitenflächen 14 der äußeren Blendenblätter 4 und 4' angefügt sind.

Die **Fig. 3c** zeigt die beiden äußeren Blendenblätter 4 und 4' geschnitten, da sie geschlossen sind. Die anderen Blendenblätter 4, 4' sind mehr oder weniger geöffnet und bilden dadurch die Kollimatoröffnung 18. Eine entsprechende Ausgestaltung der Blendenblätter 4, 4' war im Stand der Technik bereits bekannt, hat jedoch zu Funktionsproblemen geführt, die eingangs erläutert wurden. Erst durch die erfindungsgemäße Ausgestaltung der Blendenblätter 4 und 4' ist es möglich, trotz der asymmetrischen Trapezformen 13 ein einwandfreies Funktionieren zu garantieren, ohne daß dies durch große Toleranzen erkauft werden muß oder ein weiterer Satz von Blendenblättern, der um 90° versetzt angeordnet ist, in den Strahlengang 2 eingreifen muß. Auf diese Weise ist es möglich, die in der Fig. 3b dargestellte Maßnahme und die in der Fig. 3c dargestellte Maßnahme gleichzeitig bei denselben Blendenblättern 4 und 4' vorzusehen. Darin besteht eine wesentliche Überlegenheit gegenüber dem Stand der Technik.

**Fig. 4** zeigt das Prinzip eines erfindungsgemäßen Ausführungsbeispiels des Kollimators 1. Die Blendenblätter 4 und 4' bestehen aus hinteren Teilstücken 6 und 6' und vorderen Teilstücken 7 und 7'. Letztere sind als halbkreisförmige Körper 8 und 8' ausgebildet, die in korrespondierenden Ausnehmungen 9 und 9' der hinteren Teilstücke 6 und 6' der Blendenblätter 4 und 4' gelagert sind. Eine derartige Lagerung kann beispielsweise darin bestehen, daß die vorderen Teilstücke 7 und 7' an der Halbkreisform eine Nut 56 aufweisen, in die die hinteren Teilstücke 6 und 6' mit einer entsprechenden Abstufung im Bereich der korrespondierenden Ausnchmungen 9 und 9' derart eingreifen, daß die volle Materialstärke gewährleistet ist. Für eine unverlierbare Lagerung dieser vorderen Teilstücke 7 und 7' sind Haltezapfen 49 vorgeschen, die in entsprechenden Langlöchern 50 laufen. Die Länge der Langlöcher 50 entspricht dem Stellbereich. Werden die Blendenblätter 4 und 4' entsprechend den Pfeilen 48 verschoben, so werden gleichzeitig die vorderen Teilstücke 7 und 7' um eine gedachte Drehachse 36 derart gedreht, daß die Vorderkanten 5 und 5' immer parallel zu den Strahlen 2 ausgerichtet sind. Dies bedeutet, daß die Vorderkanten 5 und 5' im Bereich der Mittellinie 17 der möglichen Kollimatoröffnung 18 senkrecht sind und bei Entfernung von dieser Mittellinie 17 in der einen oder anderen Richtung derart ausgerichtet werden, daß sie zur Strahlungsquelle 3 weisen. Um diese Stellbewegungen zu gewährleisten, müssen die vorderen Enden 12 der hinteren Teilstücke 6 und 6' entsprechend zurückversetzt sein, derart, daß sich die Vorderkanten 5 und 5' erst bei maximaler Verstellung im Bereich dieser vorderen Enden 12 befinden. Zweckmäßigerweise ist die Höhe 12 der hinteren Teilstücke 6 und 6' ungefähr so groß wie der Durchmesser 11 der halbkreisförmigen Körper 8 und 8'. Dadurch ist immer dieselbe Materialstärke gewährleistet, und diese Ausführungsform hat auch den Vorteil, daß die vorderen Teilstücke 7 und 7' oben wie unten immer dieselbe Höhe bilden, wie die hinteren Teilstücken 6 und 6'.

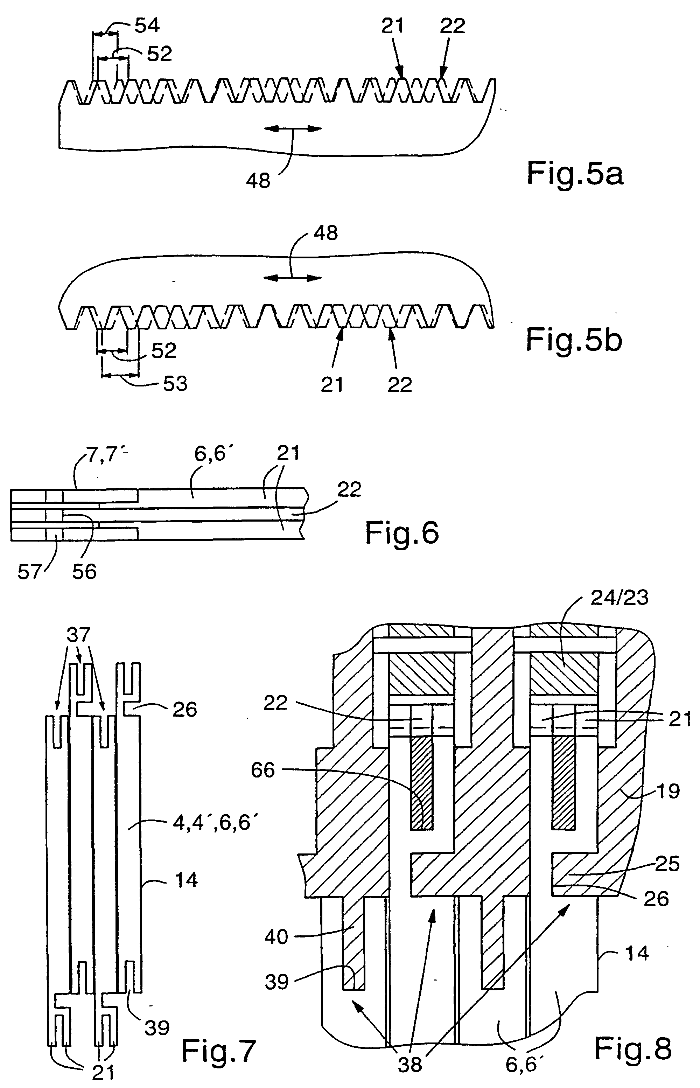

Die **Fig. 4** zeigt weiterhin ein Getriebe für die Blendenblätter 4 und 4', welches gewährleistet, daß die Vorderkanten 5 und 5' in jeder Position der Blendenblätter 4 und 4' richtig ausgerichtet sind. Dies erfolgt durch eine mechanische Zwangskopplung, die darin besteht, daß ein Antriebszahnrad 23 sowohl in eine Blendenzahnstange 21 als auch in eine Vorderkantenzahnstange 22 eingreift, wobei diese Zahnstangen 21 und 22 derart unterschiedliche Teilungen 52, 53 oder 54 (siehe Fig. 5a und b) aufweisen, daß die erforderlichen unterschiedlichen Stellbewegungen erzielt sind. Selbstverständlich muß dabei gewährleistet sein, daß die unterschiedlichen Teilungen 52, 53 oder 54 im Toleranzbereich der Verzahnung mit dem Antriebszahnrad 23 liegen, so daß keine Verklemmung eintreten kann. Der Pfeil 51 zeigt die Drehrichtung der Antriebszahnräder 23 und die Pfeile 48 die durch diese Antriebsrichtung hervorgerufenen Stellbewegungen der Blendenblätter 4 und 4'. Dabei nimmt in der Figur 4 das Blendenblatt 4 die maximale Öffnungsstellung 64 und das Blendenblatt 4' den maximalen Overtravel 63 ein. Letzteres ist die maximale Überschreitung der Mittellinie 17. Durch diesen Overtravel ist es möglich, durch die Kollimatoröffnung 18 jede Gestalt eines Tumors 20 nachzubilden, die in der Größenordnung bis zur maximalen Kollimatoröffnung 18 liegt.

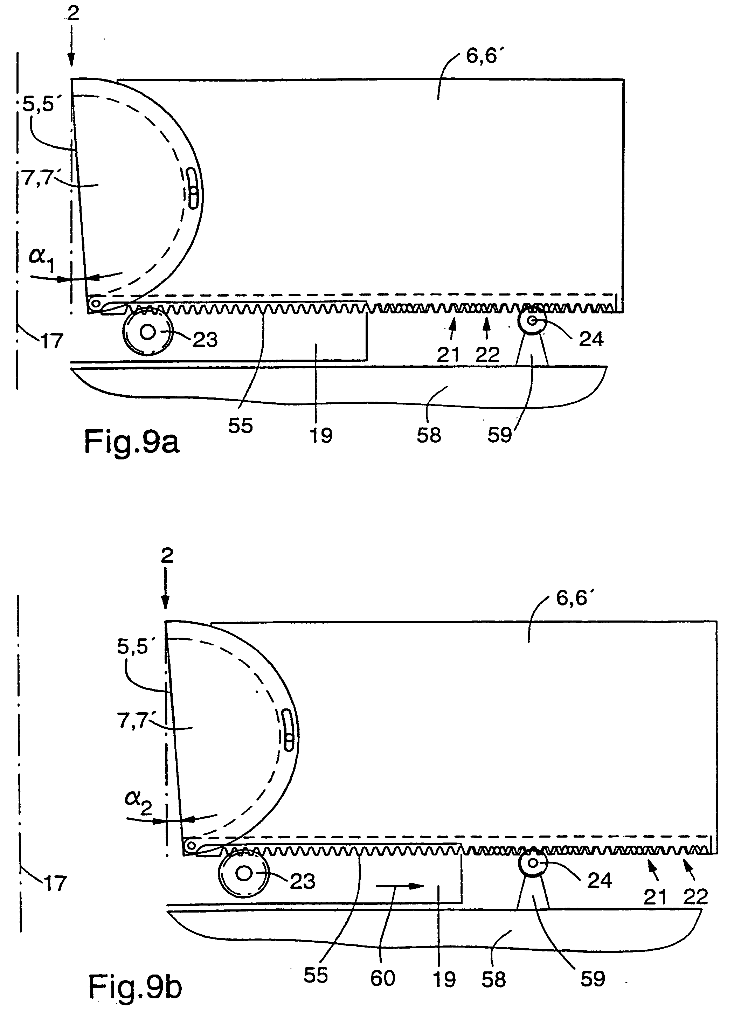

Die Anordnung der Antriebe in der Fig. 4 ist lediglich ein Beispiel, sie erfolgt am unteren Ende der Blendenblätter 4 und 4'. Es ist ebenso möglich, diese Antriebe 23, 21, 22 im oberen Bereich anzuordnen oder wechselweise an einem Blendenblatt 4 oder 4' unten und am angrenzenden Blendenblatt 4 oder 4' oben, um dadurch mehr Raum für die Anordnung dieser Antriebe zu erzielen. Bei der Ausgestaltung der **Fig. 4** sind die Zähne der Blendenzahnstange 21 in eine Längskante 37 (siehe Fig. 7) eines hinteren Teilstücks 6 oder 6' eingefräst. Diese eingefräste Blendenzahnstange 21 weist in der Mitte eine Nut 66 auf, in der sich die Vorderkantenzahnstange 22 befindet (Fig. 8).

Diese ist mittels einer Anlenkung 57 mit dem vorderen Teilstück 7, 7' schwenkbar verbunden und kann dadurch die Stellbewegung vermitteln. Da die Blendenzahnstange 21 und die Vorderkantenzahnstange 22 unterschiedliche Teilungen 52, 53, 54 aufweisen, sorgt das Antriebszahnrad 23 für unterschiedliche Vorschübe. Es ist möglich, mit Hilfe der Teilungsunterschiede die Vorschubsdifferenz zu bestimmen.

Die unterschiedlichen Zahnteilungen 52, 53, 54 sind in **Fig. 5a** und **5b** dargestellt. Die **Fig. 5a** zeigt die Teilungen 52, 54 von Blendenzahnstange 21 und Vorderkantenzahnstange 22, wenn diese oberhalb der Blendenblätter 4 und 4' angeordnet sind. Für diesen Fall ist die Teilung 52 der Blendenzahnstange 21 größer als die Teilung 54 der Vorderkantenzahnstange 22. Dadurch wird erreicht, daß die Blendenzahnstange 21 einen größeren Vorschub erhält als die Vorderkantenzahnstange 22. Wird also das hintere Teilstück 6, 6' in Richtung des Doppelpfeils 48 bewegt, so ist dessen Stellbewegung etwas größer als die der oberhalb angeordneten Vorderkantenzahnstange 22, und das vordere Teilstück 7, 7' wird derart gedreht, daß die Vorderkante 5, 5' parallel zu den Strahlen 2 verläuft. Diese Ausrichtung ist in jeder Position gewährleistet, auch wenn die Mittellinie 17 überschritten wird. Die **Fig. 5b** zeigt, daß die Teilung 52 der Blendenzahnstange 21 bei einer Anordnung unterhalb der Blendenblätter 4 und 4' kleiner ist als die Teilung 53 der Vorderkantenzahnstange 22. Die Funktion ist die gleiche, wie soeben beschrieben, mit dem Unterschied, daß bei dieser Anordnung für eine entsprechende Ausrichtung der Vorderkanten 5 und 5' der Vorschub der Vorderkantenzahnstange 22 größer sein muß als die der Blendenzahnstange 21.

Selbstverständlich sind auch andere Anordnungen möglich. Beispielsweise können die Zahnstangen 21 und 22 auch an rückwärtigen Verlängerungen der Blendenblätter 4 oder 4' angeordnet sein, und es ist auch möglich, ein gesondertes Antriebszahnrad 23 vorzusehen, um die Zuordnung der Vorschübe für die beiden Zahnstangen 21 und 22 durch ein leerlaufendes Zahnrad 24 zu gewährleisten.

**Fig. 6** zeigt eine Anlenkung eines vorderen Teilstücks 7 oder 7' an ein hinteres Teilstück 6 oder 6' eines Blendenblattes 4 oder 4'. Dabei ist zu sehen, wie die Vorderkantenzahnstange 22 in einer Nut 66 geführt ist, die in der Mitte der Blendenzahnstange 21 eingefräst wurde. Dadurch sind beide Verzahnungen auf gleicher Höhe, damit ein Zahnrad 23 oder 24 in beide Verzahnungen eingreifen kann. Da die Blendenzahnstange 21 in eine Längskante 37 eines hinteren Teilstücks 6 oder 6' unmittelbar eingefräst ist, wird diese Stellbewegung unmittelbar auf dieses hintere Teilstück 6 oder 6' übertragen. Zur Durchführung der Stellbewegung der vorderen Teilstücke 7 und 7' ist die Vorderkantenzahnstange 22 durch eine Anlenkung 57 an das vordere Teilstück 7 oder 7' schwenkbar angebracht und vermittelt dadurch die Stellbewegung.

**Fig. 7** zeigt eine versetzte Anordnung der hinteren Teilstücke 6 oder 6' der Blendenblätter 4 oder 4'. Diese versetzte Anordnung dient der Unterbringung von Führungen 38, welche mittels Nuten 26 und 39 erfolgt. Derartige Nuten 26, 39 können entweder in die Seitenflächen 14 oder in die Längskanten 37 eingefräst sein.

**Fig. 8** zeigt die Anordnung derartiger Führungen 38 sowie die Anordnung eines Antriebszahnrades 23 oder eines Zahnrades 24 sowie der Blendenzahnstange 21 und der Vorderkantenzahnstange 22. Eine erste Führung 38 besteht darin, daß in die Längskante 37 des hinteren Teilstücks 6 oder 6' eine Nut 39 eingefräst ist, in der ein Führungselement 40 des Kollimatorblocks 19 läuft. Eine weitere Führung 38 besteht aus einer Führungsnut 26, die sich in der Seitenflüche 14 eines hinteren Teilstücks 6 oder 6' befindet. Auch in diese Führungsnut 26 greift ein Führungselement 25 des Kollimatorblocks 19 ein. Dabei ist die Führungsnut 26 an dem Ende des hinteren Teilstücks 6 oder 6' angeordnet, an dem sich auch die Blendenzahnstange 21 befindet. Die Blendenzahnstange 21 ist in eine Längskante 37 des hinteren Teilstücks 6 oder 6' eingefräst. Im mittleren Bereich dieser Blendenzahnstange 21 befindet sich eine Nut 66, in welcher die Vorderkantenzahnstange 22 derart gelagert ist, daß in deren Verzahnung genauso wie in die Verzahnung der Blendenzahnstange 21 ein Zahnrad 24 oder 23 eingreift. Dabei werden aufgrund der unterschiedlichen Teilungen verschiedene Vorschübe erzielt, wie dies oben beschrieben ist.

Die **Fig. 9a** und **9b** zeigen ein zweites Ausführungsbeispiel der Erfindung, das sich vom ersten Ausführungsbeispiel dadurch unterscheidet, daß sich im vorderen Bereich des hinteren Teilstücks 6 oder 6' eine Antriebszahnstange 55 und ein Antriebszahnrad 23 befindet und die Blendenzahnstange 21 sowie die Vorderkantenzahnstange 22 im hinteren Bereich angeordnet sind. Dabei greift ein lose laufendes Zahnrad 24 in beide Zahnstangen 21 und 22 ein, um der Vorderkantenzahnstange 22 den abweichenden Vorschub zu vermitteln. In diesem Ausführungsbeispiel ist das Antriebszahnrad 23 in einem Kollimatorblock 19 oder in einer Kollimatorblockhälfte gelagert, der/die gegenüber einem Grundgestell 58 verschiebbar ist. Das weitere Zahnrad 24 ist über eine Lagerung 59 mit dem Grundgestell 58 verbunden. Der Zweck dieser Anordnung besteht darin, daß die einmal in der richtigen Ausrichtung justierten Vorderkanten 5 und 5' auch dann parallel zu den Strahlen 2 ausgerichtet bleiben, wenn der gesamte Kollimatorblock 19 gegenüber der Strahlungsquelle 3 verschoben wird oder wenn zwei Kollimatorblockhälften zur Vergrößerung der Kollimatoröffnung auseinandergeschoben werden. Dies ist dadurch gezeigt, daß sich der Kollimatorblock 19 in der **Fig. 9a** in einer ersten Position zur Mittellinie 17 befindet und in der **Fig. 9b** in einer zweiten Position, wobei er in Richtung des Pfeils 60 verschoben wurde. Durch diese Verschiebung hat sich mit Hilfe der dargestellten Mechanik der Winkel α₂ des vorderen Teilstücks 7 oder 7' derart verändert, daß die Vorderkanten 5 oder 5' auch in der neuen Position parallel zu den Strahlen 2 verlaufen. Es ist ersichtlich, daß die Vorderkante 5 oder 5' in der **Fig. 9b** einen größeren Abstand zur Mittellinie 17 aufweist als in der **Fig. 9a** und sich der Winkel von α₁ durch das Verschieben zu α₂ vergrößert hat.

Die Antriebszahnstange 55 im vorderen Bereich kann dabei identisch mit der Verzahnung der Blendenzahnstange 21 sein oder eine andere Teilung oder Zahngröße aufweisen. Auf jeden Fall ist erforderlich, daß die Vorderkantenzahnstange 22 in diesem Bereich keine Zähne aufweist und so tief in der Nut 66 liegt, daß das Antriebszahnrad 23 in der Antriebszahnstange 55 laufen kann und die Vorderkantenzahnstange 22 in diesem Bereich frei verschiebbar ist.

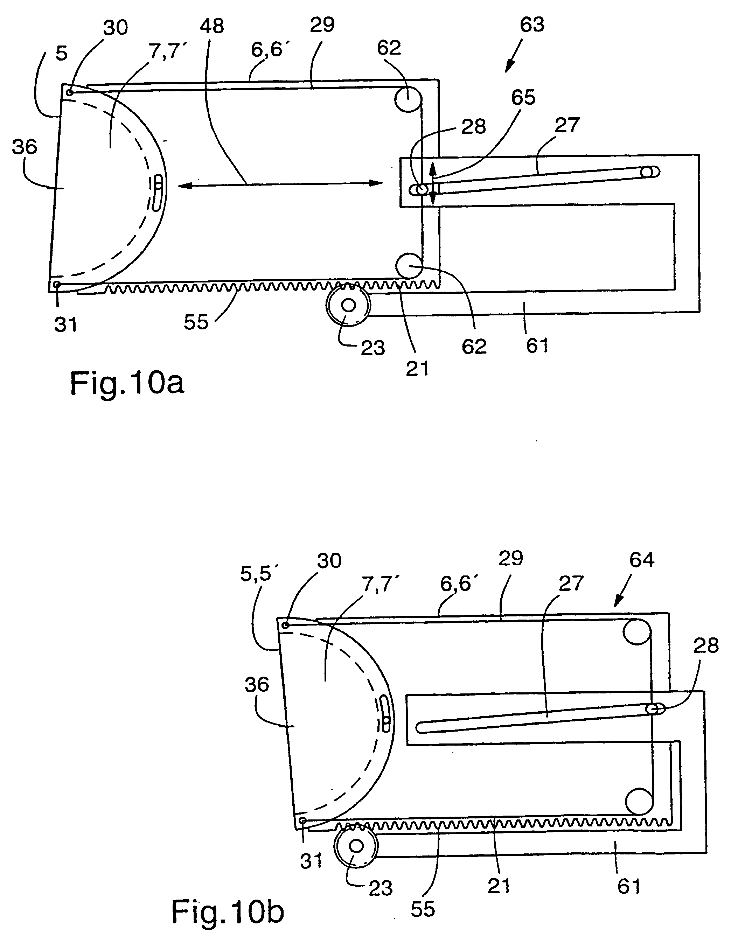

Die **Fig. 10a** und **10b** zeigen ein drittes Ausführungsbeispiel, bei dem die Stellbewegung der vorderen Teilstücke 7 und 7' durch eine Kulissensteuerung bewirkt wird. Auch bei diesem Ausführungsbeispiel ist an den hinteren Teilstücken 6 oder 6' eine Antriebszahnstange 55 angebracht, welche mittels eines Antriebszahnrads 23 der Stellbewegung dieses hinteren Teilstücks 6 oder 6' dient. Der Erzeugung der Stellbewegung des vorderen Teilstücks 7 oder 7' dient eine Kulissenführung 27, die durch eine feste Verbindung 61 mit dem Antriebszahnrad 23 verbunden ist. In dieser Kulissenführung 27 läuft ein Gleitstück 28, welches an einem Seilzug 29 befestigt ist. Dieser Seilzug 29 ist mit einem Ende 30 oberhalb der gedachten Drehachse 36 am vorderen Teilstück 7 oder 7' befestigt und mit dem anderen Ende 31 unterhalb dieser gedachten Drehachse 36.

Die **Fig. 10a** und **10b** zeigen den möglichen Stellbereich, wobei die Fig. 10a die Position des maximalen Overtravels 63 zeigt und die Fig. 10b die maximale Öffnung 64. Wird durch das Antriebszahnrad 23 dem hinteren Teilstück 6 oder 6' die Stellbewegung 48 vermittelt, so wird das Gleitstück 28 durch die Kulissenführung 27 in Richtung des Pfeils 65 verschoben.

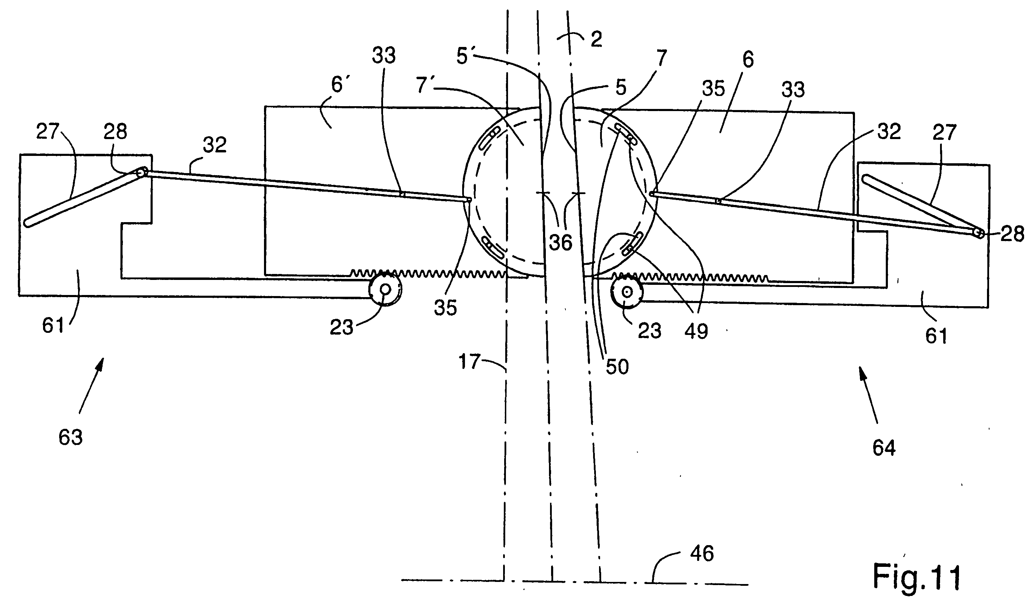

**Fig. 11** zeigt ein viertes Ausführungsbeispiel, das sich von dem dritten Ausführungsbeispiel dadurch unterscheidet, daß sich das Gleitstück 28 am Ende eines doppelarmigen Hebels 32 befindet. Der Hebel 32 ist mittels einer Drehachse 33 an dem hinteren Teilstück 6 oder 6' angelenkt. Am vorderen Ende 35 ist der doppelarmige Hebel 32 schwenkbar an dem vorderen Teilstück 7 oder 7' angelenkt und zwar in dem hinteren Bereich, also von der gedachten Drehachse 36 entfernt.

Bei diesem Ausführungsbeispiel wird der doppelarmig Hebel 32 durch die Kulissenführung 27 geschwenkt und führt dabei die Stellbewegung aus, welche zur entsprechenden Ausrichtung der Vorderkanten 5 oder 5' der Blendenblätter 4 oder 4' führt. Für die Unterbringung des doppelarmigen Hebels 32 muß allerdings eine gewisse Aussparung in den hinteren Teilstücken 6 oder 6' vorgesehen sein. Die Fig. 11 zeigt auf der einen Seite den maximalen Overtravel 63 und auf der anderen Seite die maximale Öffnung 64.

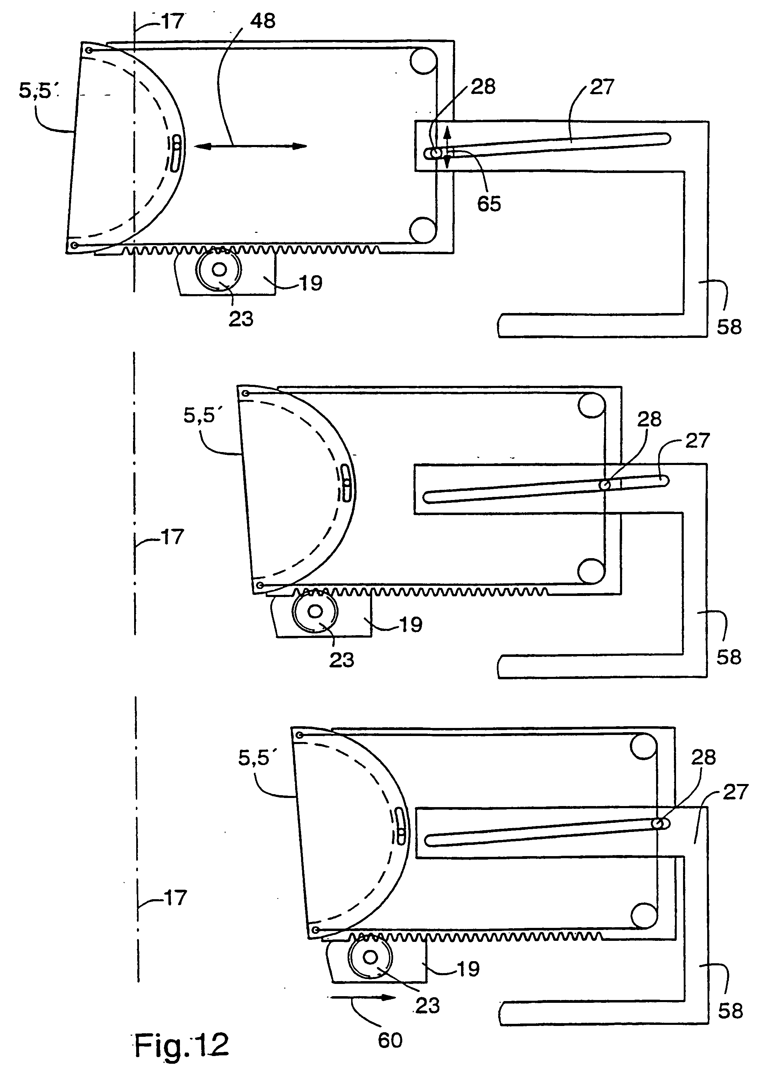

**Fig. 12** zeigt eine weitere Ausgestaltungsform des Ausführungsbeispiels der Fig. 10a und b. Der Unterschied besteht darin, daß die Kulissenführungen 27 mit dem Grundgestell 58 und die Antriebszahnräder 23 mit dem Kollimatorblock 19 oder Kollimatorblockhälften verbunden sind. Auf diese Weise läßt sich die Kollimatoröffnung 18 obenfalls vergrößern. Die Kulissenführungen 27 müssen dann eine Länge aufweisen, welche dem gesamten Stellweg entspricht, also dem Stellweg der Blendenblätter 4, 4' und dem Stellweg der Kollimatorblockhälften. Auch die in Fig. 11 gezeigte Ausführungsform läßt sich in entsprechender Weise abwandeln.

Die dargestellten Ausgestaltungen sind selbstverständlich nur beispielhaft, insbesondere bezüglich der Zwangskopplung, jedoch auch bezüglich der Antriebe und der Ausgestaltung der beiden Teilstücke der Blendenblätter sind weitere Ausführungsformen denkbar.

### Kollimator zum Begrenzen eines Bündels energiereicher Strahlen

### Bezugszeichenliste

- 1: Kollimator
- 2: Strahlen
- 3: Strahlungsquelle
- 4,4': Blendenblätter
- 5, 5': Vorderkanten der Blendenblätter
- 6, 6': hinteres Teilstück der Blendenblätter
- 7, 7': vorderes Teilstück der Blendenblätter
- 8, 8': halbkreisförmiger Körper
- 9, 9': korrespondierende Ausnehmungen
- 10: Höhe des hinteren Teilstücks
- 11: Durchmesser des halbkreisförmigen Körpers
- 12: vordere Enden des hinteren Teilstücks
- 13: asymmetrische Trapezformen
- 14: Seitenflächen der Blendenblätter
- 15: Innenflächen der seitlichen Begrenzungen
- 16: seitliche Begrenzungen der möglichen Kollimatoröffnung
- 17: Mittellinie der möglichen Kollimatoröffnung
- 18: Kollimatoröffnung
- 19: Kollimatorblock
- 20: Bestrahlungsobjekt (Tumor)
- 21: Blendenzahnstange
- 22: Vorderkantenzahnstange
- 23: Antriebszahnrad
- 24: Zahnrad
- 25: Führungselement
- 26: Führungsnut
- 27: Kulissenführung
- 28: Gleitstück
- 29: Seilzug
- 30: ein Ende des Seilzugs
- 31: anderes Ende des Seilzugs
- 32: doppelarmiger Hebel
- 33: Drehachse des doppelarmigen Hebels
- 34: hinteres Ende des doppelarmigen Hebels
- 35: vorderes Ende des doppelarmigen Hehels
- 36: gedachte Drehachse
- 37: Längskante des hinteren Teilstücks
- 38: Führung
- 39: Nut
- 40: Führungselement
- 41: Gantry
- 42: Behandlungstisch
- 43: Linearbeschleuniger
- 44: horizontale Rotationsachse der Gantry
- 45: Rotationsachse des Behandlungstisches
- 46: Patient
- 47: Halbschatten
- 48: Pfeil: Stellbewegung der Blendenblätter
- 49: Haltezapfen
- 50: Langloch
- 51: Pfeil: Drehrichtung des Antriebszahnrades
- 52: Teilung der Blendenzahnstange
- 53: Teilung einer unterhalb einer Blende angeordneten Vorderkantenzahnstange
- 54: Teilung einer oberhalb einer Blende angeordneten Vorderkantenzahnstange
- 55: Antriebszahnstange
- 56: Nut zur Führung eines vorderen Teilstücks in einem hinteren Teilstück
- 57: Anlenkung der Vorderkantenzahnstange an das vordere Teilstück
- 58: Grundgestell
- 59: Lagerung des weiteren Zahnrads
- 60: Pfeil: Verschiebung des Kollimatorblocks
- 61: feste Verbindung: Kulissenführung-Antriebszahnrad
- 62: Umlenkrollen
- 63: maximaler Overtravel eines Blendenblattes
- 64: maximale Öffnung eines Blendenblattes
- 65: Pfeil: Stellbewegung des Gleitstücks 28
- 66: Nut

## Patentansprüche

1. Multileaf Kollimator (1) zum Begrenzen eines Bündels energiereicher Strahlen (2), das von einer im wesentlichen punktförmigen Strahlungsquelle (3) ausgehend auf ein Behandlungsobjekt (20) gerichtet ist und insbesondere der stereotaktischen Konformationsbestrahlung von Tumoren dient, wobei der Kollimator (1) eine Vielzahl einander gegenüberliegender Blendenblätter (4, 4') aus strahlungsabsorbierendem Material enthält, welche durch Antriebe derart in den Strahlengang bringbar sind, daß dieser bezüglich seiner Kontur beliebig begrenzbar ist und dabei die Vorderkanten (5, 5') der Blendenblätter (4, 4') immer parallel zum Strahlengang ausgerichtet sind,
**dadurch gekennzeichnet,**
**daß** die Blendenblätter (4, 4') aus einem geradlinig verschiebbar gelagerten hinteren Teilstück (6, 6') und einem an diesem angelenkten vorderen Teilstück (7, 7') bestehen, wobei das vordere Teilstück (7, 7') eines jeden Blendenblattes (4, 4') durch Antriebsmittel eine derartige, der jeweiligen Position des jeweiligen hinteren Teilstücks (6, 6') zugeordnete Stellbewegung erfährt, daß die Vorderkanten (5, 5') immer parallel zum Strahlengang ausgerichtet sind und, daß die Anlenkung des vorderen Teilstücks (7, 7') an das hintere Teilstück (6, 6') derart erfolgt, daß **dadurch** keine nennenswerte Unterbrechung des Volumens des strahlunssabsorbierenden Materials eintritt.

2. Kollimator nach Anspruch 1,
**dadurch gekennzeichnet,**
**daß** die Antriebsmittel derart ausgebildet sind, daß eine mechanische Zwangskopplung zu jeder Position des hinteren Teilstücks (6, 6') die zugehörige Ausrichtung des vorderen Teilstücks (7, 7') und damit der Vorderkanten (5, 5') garantiert.

3. Kollimator nach Anspruch 1 oder 2,
**dadurch gekennzeichnet,**
**daß** die vorderen Teilstücke (7, 7') im wesentlichen halbkreisförmige Körper (8, 8') sind, die in korrespondierenden Ausnehmungen (9, 9') am vorderen Ende der hinteren Teilstücke (6, 6') unverlierbar gelagert sind, wobei die Stellbewegung eine Schwenkung um die im Mittelpunkt der Kreislinie liegende gedachte Drehachse (36) ist.

4. Kollimator nach Anspruch 3,
**dadurch gekennzeichnet,**
**daß** die Höhe (10) des hinteren Teilstücks (6, 6') im wesentlichen dem Durchmesser (11) des halbkreisförmigen Körpers (8, 8') entspricht und die vorderen Enden (12) des hinteren Teilstücks (6, 6') derart zurückversetzt sind, daß jede erforderliche Schrägstellung der Vorderkanten (5, 5') der Blendenblätter (4, 4') möglich ist.

5. Kollimator nach einem der Ansprüche 1 bis 4,
**dadurch gekennzeichnet,**
**daß** die Querschnitte der Blendenblätter (4, 4') eine derartige asymmetrische Trapezform (13) aufweisen, daß deren Seitenflächen (14) in etwa parallel zum Strahlengang verlaufen und daß die Innenflächen (15) der an die äußeren Blendenblätter anschließenden seitlichen Begrenzungen (16) derart schräg verlaufen, daß sie an diese äußeren Blendenblätter (4, 4') lückenfrei anschließen.

6. Kollimator nach Anspruch 5,
**dadurch gekennzeichnet,**
**daß** die vorderen Teilstücke (7, 7') soviel seitliches Spiel aufweisen, daß deren Stellbewegung trotz der Trapezform (13) möglich ist.

7. Kollimator nach einem der Ansprüche 1 bis 6,
**dadurch gekennzeichnet,**
**daß** die Blendenblätter (4, 4') über die Mittellinie (17) der möglichen Kollimatoröffnung (18) hinaus verschiebbar sind.

8. Kollimator nach einem der Ansprüche 1 bis 7,
**dadurch gekennzeichnet,**
**daß** jedes Blendenblatt (4 oder 4') einen steuerbaren Einzelantrieb aufweist.

9. Kollimator nach einem der Ansprüche 1 bis 8,
**dadurch gekennzeichnet,**
**daß** die Steuerung des Kollimators (1) beim Betrieb desselben über einen Rechner erfolgt, der die Kontur und Lage der Kollimatoröffnung (18) dem Bestrahlungsobjekt (20) in der jeweiligen Bestrahlungsrichtung anpaßt, wobei der Rechner die Daten von einer Vorrichtung zur Erfassung der Gestalt des Bestrahlungsobjektes (20) erhält und eine Kontrolleinrichtung das Ergebnis der Stellbewegung überprüft.

10. Kollimator nach einem der Ansprüche 1 bis 9,
**dadurch gekennzeichnet,**
**daß** die Blendenblätter (4, 4') in einem Kollimatorblock (19) oder Kollimatorblockhälften gelagert sind, der/die zur Positionierung der Kollimatoröffnung (18) relativ zu Bestrahlungsobjekt (20) und zur Strahlungsquelle (3) dient / dienen.

11. Kollimator nach Anspruch 10,
**dadurch gekennzeichnet,**
**daß** der Kollimatorblock (19) auf einem Gantry (41) gelagert ist, wobei eine derartige Relativbewegung zwischen Kollimatorblock (19) und Patienten (46) möglich ist, daß der Patient (46) unter Anpassung der Kollimatoröffnung (18) an die Gestalt des Bestrahlungsobjektes (20) von allen Seiten bestrahlbar ist.

12. Kollimator nach Anspruch 2 oder einem der Ansprüche 3 bis 11 mit Anspruch 2,
**dadurch gekennzeichnet,**
**daß** die Zwangskopplung des Antriebs der hinteren Teilstücke (6, 6') der Blendenblätter (4, 4') mit dem Stellantrieb für die vorderen Teilstücke (7, 7') über Getriebe erfolgt.

13. Kollimator nach Anspruch 12,
**dadurch gekennzeichnet,**
**daß** die Getriebe für die Blendenblätter (4, 4') wechselweise bei einem Blendenblatt (4, 4') oberhalb und beim angrenzenden Blendenblatt (4, 4') unterhalb angeordnet sind.

14. Kollimator nach Anspruch 12 oder 13,
**dadurch gekennzeichnet,**
**daß** der Stellantrieb für die vorderen Teilstücke (7, 7') derart ausgestaltet ist, daß er letztere sowohl bei einer individuellen Verstellung der Blendenblätter (4, 4') als auch bei einer Verstellung aller (4, 4') oder eines Teils der Blendenblätter (4 oder 4') gegenüber der Strahlungsquelle (3) ausrichtet.

15. Kollimator nach einem der Ansprüche 12 bis 14,
**dadurch gekennzeichnet,**
**daß** dem hinteren Teilstück (6, 6') eine Blendenzahnstange (2 1) zugeordnet ist, in die ein Antriebszahnrad (23) eingreift.

16. Kollimator nach Anspruch 15,
**dadurch gekennzeichnet,**
**daß** die dem hinteren Teilstück (6, 6') zugeordnete Blendenzahnstange (21) als Verzahnung einer Längskante (37) ausgeführt ist.

17. Kollimator nach Anspruch 16,
**dadurch gekennzeichnet,**
**daß** im Bereich der Verzahnung der Längskante (37) das angrenzende hintere Teilstück (6, 6') derart in der Höhe versetzt im Kollimatorblock (19) gelagert ist, daß oberhalb der Verzahnung seitlich ein mit dem Kollimatorblock (19) in Verbindung stehendes Führungselement (25) in eine Führungsnut (26) des hinteren Teilstücks (6, 6') eingreift.

18. Kollimator nach einem der Ansprüche 15 bis 17,
**dadurch gekennzeichnet,**
**daß** am vorderen Teilstück (7, 7') außerhalb von dessen Drehachse (36) eine Vorderkantenzahnstange (22) angelenkt ist, in die ein Zahnrad (23 oder 24) eingreift, wobei ein gegenüber dem hinteren Teilstück (6, 6') abweichender Stellweg erzielt wird, derart, daß die entsprechende Ausrichtung der Vorderkante (5, 5') erfolgt.

19. Kollimator nach Anspruch 18,
**dadurch gekennzeichnet,**
**daß** die Blendenzahnstange (21) und die Vorderkantenzahnstange (22) an einer Längskante (37) des hinteren Teilstücks (6, 6') angeordnet sind und zur Erzielung des abweichenden Stellwegs eine unterschiedliche Teilung (52, 53, 54) aufweisen, wobei ein Zahnrad (23 oder 24) in beide Zahnstangen (21, 22) eingreift und dabei der Teilungsunterschied im Toleranzhereich der Verzahnung liegt.

20. Kollimator nach Anspruch 19,
**dadurch gekennzeichnet,**
**daß** für ein unterhalb einer Blende (4, 4') angeordnetes Getriebe die Teilung (53) der Vorderkantenzahnstange (22) größer ist als die Teilung (52) der Blendenzahnstange (21).

21. Kollimator nach Anspruch 19 oder 20,
**dadurch gekennzeichnet,**
**daß** für ein oberhalb einer Blende (4, 4') angeordnetes Getriebe die Teilung (54) der Vorderkantenzahnstange (22) kleiner ist als die Teilung (52) der Blendenzahnstange (21).

22. Kollimator nach einem der Ansprüche 18 bis 21,
**dadurch gekennzeichnet,**
**daß** die Zahnräder (23 oder 24) in einem Grundgestell (58) gelagert sind.

23. Kollimator nach einem der Ansprüche 18 bis 22,
**dadurch gekennzeichnet,**
**daß** die Zahnräder (24) gleichzeitig als Antriebszahnräder (23) dienen und im Kollimatorblock (19) gelagert sind.

24. Kollimator nach Anspruch 14 mit einem der Ansprüche 19 bis 23,
**dadurch gekennzeichnet,**
**daß** die Antriebszahnräder (23), welche in die Antriebszahnstangen (55, 21 oder 22) eingreifen, in einem verschiebbaren Kollimatorblock (19) oder zwei verschiebbaren Kollimatorblockhälften und weitere Zahnräder (24), welche in die Blendenzahnstangen (21) und die Vorderkantenzahnstangen (22) eingreifen, auf einem Grundgestell gelagert sind, auf dem der Kollimatorblock (19) insgesamt oder als Kollimatorblockhälften - für je einen Teil der Blendenblätter (4 oder 4') - verschiebbar sind.

25. Kollimator nach einem der Ansprüche 14 bis 17,
**dadurch gekennzeichnet,**
**daß** Kulissensteuerungen der Stellbewegung der vorderen Teilstücke (7, 7') dienen.

26. Kollimator nach Anspruch 25, mit Anspruch 15,
**dadurch gekennzeichnet,**
**daß** die Kulissenführungen (27) mit der Lagerung der Antriebszahnräder (23) in fester Verbindung (61) und die Gleitstücke (28) der Kulissensteurung mit dem vorderen Teilstücken (7, 7') in Wirkverbindung stehen.

27. Kollimator nach Anspruch 25 mit Anspruch 15,
**dadurch gekennzeichnet,**
**daß** die Kulissenführungen (27) mit einem Grundgestell (58) in fester Verbindung stehen und verschiebbare Kollimatorblockhälften - für je einen Teil der Blendenblätter (4 oder 4') - mit den Lagerungen der Antriebszahnräder (23) in fester Verbindung stehen.

28. Kollimator nach Anspruch 26 oder 27,
**dadurch gekennzeichnet,**
**daß** das Gleitstück (28) an einem Seilzug (29) befestigt ist, der zum vorderen Teilstück (7, 7') geführt und mit einem Ende (30) oberhalb und dem anderen Ende (31) unterhalb der gedachten Drehachse (36) der vorderen Teilstücks (7, 7') befestigt ist.

29. Kollimator nach Anspruch 26 oder 27,
**dadurch gekennzeichnet,**
**daß** das Gleitstück (28) an einem hinteren Ende (34) eines doppelarmigen Hebels (32) befestigt ist, wobei der Hebel (32) mit seiner Drehachse (33) auf dem hinteren Teilstück (6, 6') gelagert und mit seinem vorderen Ende (35) im hinteren Bereich des vorderen Teilstücks (7, 7') gelagen ist.

30. Kollimator nach einem der Ansprüche 1 bis 29,
**dadurch gekennzeichnet,**
**daß** an mindestens einer Längskante (37) eines hinteren Teilstücks (6, 6') eine Führung (38) vorgesehen ist.

31. Kollimator nach Anspruch 30,
**dadurch gekennzeichnet,**
**daß** die Führung (38) aus einer Nut (39) in der Längskante (37) besteht, in der ein Führungselement (40) gleitet.

32. Kollimator nach einem der Ansprüche 1 bis 31,
**dadurch gekennzeichnet,**
**daß** die Blendenblätter (4, 4') als Kompensatormittel zur Erzeugung unterschiedlicher Strahlungsintensität dienen, indem sie während der Bestrahlung zeitweise in die Kollimatoröffnung (18) einfahrbar sind.

## Claims

1. A multileaf collimator (1) for defining a bundle of high-energy radiation (2) which originates from a substantially punctiform radiation source (3) and is directed onto an object to be therapied (20) and is used, in particular, for stereotactic conformation radiation therapy of tumors, which collimator (1) contains a large number of opposing aperture leaves (4, 4') of radiation absorbing material, which can be driven to enter the optical path in such a manner that the latter can be delimited to any desired extent with regard to its contour, while the front edges (5, 5') of said aperture leaves (4, 4') are always oriented parallel to the optical path,
**characterized in that**
said aperture leaves (4, 4) consist of a rear section (6, 6') mounted for linear displacement and a front section (7, 7') hingedly attached thereto, wherein said front section (7, 7') of each aperture leaf (4, 4') is actuated by driving means to assume a position relative to said respective rear section (6, 6') such that said front edges (5, 5') are always parallel to the optical path, and wherein said front section (7, 7) is hinged to said rear section (6, 6) in such a manner that it causes no appreciable volume loss of the radiation absorbing material.

2. A collimator as defined in claim 1, **characterized in that** said driving means are designed in such a manner that mechanical forced coupling to each position of said rear section (6, 6') ensures correct alignment of said front section (7, 7') and thus of its front edges (5, 5').

3. A collimator as defined in claim 1 or claim 2, **characterized in that** said front sections (7, 7') are substantially semicircular bodies (8, 8') which are captively mounted in corresponding recesses (9, 9') at the front end of said rear sections (6, 6'), whilst actuation thereof is effected in the form of a rotational movement about the imaginary axis of rotation (36) located at the center of the arc defined by said semicircular bodies.

4. A collimator as defined in claim 3, **characterized in that** the height (10) of said rear section (6, 6') is substantially equal to the diameter (11) of said semicircular body (8, 8') and the front ends (12) of said rear section (6, 6) are out of line to such an extent that all necessary inclined positions of said front edges (5, 5') of said aperture leaves (4, 4) are possible.

5. A collimator as defined in any one of claims 1 through 4, **characterized in that** the cross-sections of said aperture leaves (4, 4') show an asymmetrical trapezoid form (3) such that their lateral surfaces (14) extend approximately parallel to the optical path and that the inner surfaces (15) of the lateral delimiters (16) adjacent to the outer aperture leaves slope in such a manner that they abut said outer aperture leaves (4, 4') without gap formation.

6. A collimator as defined in claim 5, **characterized in that** said front sections (7, 7') have sufficient lateral play to enable actuation thereof despite the trapezoid form (13).

7. A collimator as defined in any one of claims 1 through 6, **characterized in that** said aperture leaves (4, 4') can be displaced beyond the center line (17) of the possible collimator aperture (18).

8. A collimator as defined in any one of claims 1 through 7, **characterized in that** each aperture leaf (4 or 4') has an individual controllable drive.

9. A collimator as defined in any one of claims 1 through 8, **characterized in that** control of said collimator (1) during operation thereof is effected via a computer, which adapts the periphery and position of the collimator aperture (18) to the object to be irradiated (20) in the respective direction of irradiation, which computer receives data from a device for ascertaining the shape of the object to be irradiated (20), and supervisory equipment checks the result of the actuation.

10. A collimator as defined in any one of claims 1 through 9, **characterized in that** said aperture leaves (4, 4') are mounted in a collimator block (19) or collimator block halves which serve(s) to position the collimator aperture (18) relatively to the object to be irradiated (20) and to the source of radiation (3).

11. A collimator as defined in claim 10, **characterized in that** said collimator block (19) is mounted on a gantry (41) to enable relative movement between said collimator block (19) and the patient (46) such that the patient (46) can be irradiated from all sides while the collimator aperture (18) is adapted to the shape of the object to be irradiated (20).

12. A collimator as defined in claim 2 or in any one of claims 3 through 11 in conjunction with claim 2, **characterized in that** forced coupling of the drive for said rear sections (6, 6) of said aperture leaves (4, 4') with the actuator for said front sections (7, 7') is effected via gearing.

13. A collimator as defined in claim 12, **characterized in that** said gear wheels for said aperture leaves (4, 4') are alternately disposed on one aperture leaf (4, 4') above the same and on the adjacent aperture leaf (4, 4') below the same.

14. A collimator as defined in claim 12 or claim 13, **characterized in that** the actuator for said front sections (7, 7') is designed in such a manner that it aligns the latter toward the source of radiation (3) both when the aperture leaves (4, 4') are adjusted individually and when all (4, 4') or some (4 or 4') of the aperture leaves are adjusted.

15. A collimator as defined in any one of claims 12 through 14, **characterized in that** to said rear section (6, 6') there is assigned an aperture toothed rack (21) which is engaged by a driving toothed wheel (23).

16. A collimator as defined in claim 15, **characterized in** said aperture toothed rack (21) assigned to said section (6, 6') is in the form of a series of teeth provided on a longitudinal edge (37).

17. A collimator as defined in claim 16, **characterized in that** in the region of said teeth on said longitudinal edge (37) the adjacent rear section (6, 6') is mounted in the collimator block (19) out of height alignment such that a guide element (25) connected to said collimator block (19) laterally engages a guide slot (26) in said rear section (6, 6') above said teeth.

18. A collimator as defined in any one of claims 15 through 17, **characterized in that** a front edge toothed rack (22) is hingedly attached to said front section (7, 7') in a region outside its axis of rotation (36), which toothed rack is engaged by a gearwheel (23 or 24) to achieve corrective displacement deviating from said rear section (6, 6') such that the required alignment of the front edge (5, 5') is attained.

19. A collimator as defined in claim 18, **characterized in that** said aperture toothed rack (21) and said front edge toothed rack (22) are located on a longitudinal edge (37) of said rear section (6, 6') and have different pitches to achieve said deviating corrective displacement (52, 53, 54), and a gearwheel (23 or 24) engages both toothed racks (21, 22), the difference in pitch being within the tolerance range of the teeth.

20. A collimator as defined in claim 19, **characterized in that** for gearing located below an aperture leaf (4, 4') the pitch (53) of said front edge toothed rack (22) is greater than the pitch (52) of said aperture toothed rack (21).

21. A collimator as defined in claim 19 or claim 20, **characterized in that** for gearing located above an aperture leaf (4, 4) the pitch (54) of said front edge toothed rack (22) is smaller than the pitch (52) of said aperture toothed rack (21).

22. A collimator as defined in any one of claims 18 through 21, **characterized in that** said gear wheels (23 or 24) are mounted in a base frame (58).

23. A collimator as defined in any one of claims 18 through 22, **characterized in that** the gear wheels (24) at the same time serve as driving gear wheels (23) and are mounted in said collimator block (19).

24. A collimator as defined in claim 14 in conjunction with any one of claims 19 through 23, **characterized in that** said driving gear wheels (23) which mesh with said driving toothed racks (55, 21, or 22) are mounted in a displaceable collimator block (19) or two displaceable collimator block halves, and further gear wheels (24) which mesh with said aperture toothed racks (21) and said front edge toothed racks (22) are mounted on a base frame, on which said collimator block (19) as a whole or as collimator block halves - each for a portion of said aperture leaves (4 or 4') - is displaceably mounted.

25. A collimator as defined in any one of claims 14 through 17, **characterized in that** link movements serve to actuate said front sections (7, 7').

26. A collimator as defined in claim 25 in conjunction with claim 15, **characterized in that** link guides (27) are rigidly connected (61) to the bearings of the driving gear wheels (23), and sliding pieces (28) of the control link are actively connected to said front sections (7, 7).

27. A collimator as defined in claim 25 in conjunction with claim 15, **characterized in that** said link guides (27) are rigidly connected to a base frame (58), and displaceable collimator block halves - each associated with a portion of said aperture leaves (4 or 4') - are rigidly connected to the bearings of said driving gear wheels (23).

28. A collimator as defined in claim 26 or claim 27, **characterized in that** said sliding piece (28) is attached to a cable (29) which extends to said front section (7, 7') and is attached at one end (30) above, and at the other end (31) below, the imaginary rotation axis (36) of said front section (7, 7).

29. A collimator as defined in claim 26 or claim 27, **characterized in that** said sliding piece (28) is attached to a rear end (34) of a double-armed lever (32), which lever (32) is connected, via its rotation axis (33), to said rear section (6, 6') and, via its front end (35), to the rear part of said front section (7, 7').

30. A collimator as defined in any one of claims 1 through 29, **characterized in that** guide means (38) are provided on at least one longitudinal edge (37) of a rear section (6, 6').

31. A collimator as defined in claim 30, **characterized in that** said guide means (38) consist of a groove (39) in said longitudinal edge (37), in which groove a guide element (40) slides.

32. A collimator as defined in any one of claims 1 through 31, **characterized in that** said aperture leaves (4, 4') serve as compensating means for producing different radiant intensities by being moved temporarily into the collimator aperture (18) during irradiation.

## Revendications

1. Collimateur multilames (1) pour la délimitation d'un faisceau de rayons riches en énergie (2), qui est dirigé en partant d'une source de rayonnement sensiblement punctiforme sur un objet de traitement (20) et sert en particulier au rayonnement stéréotactique en conformation, le collimateur (1) contenant un grand nombre de lames d'obturateur opposées (4, 4') en matériau absorbant le rayonnement qui peuvent être amenées par des entraînements dans la trajectoire de rayon de sorte que celui-ci peut être délimité par rapport à son contour de manière illimitée et de ce fait les bords avant (5, 5') des lames d'obturateurs (4, 4') sont toujours dirigés parallèlement à la trajectoire du rayon,
**caractérisé en ce que** les lames d'obturateur (4, 4') se composent d'une partie arrière (6, 6') logée coulissante linéairement et d'une partie avant articulée (7, 7'), la partie avant (7, 7') de chaque lame d'obturateur (4, 4') atteint par des moyens d'entraînement un tel mouvement de réglage associé à la position respective de la partie arrière respective (6, 6'), de sorte que les bords avant (5, 5') sont toujours dirigés parallèlement à la trajectoire de rayon et **en ce que** l'articulation de la partie avant (7, 7') sur le morceau arrière (6, 6') s'effectue de façon qu'il ne se produise aucune interruption notable du volume du matériau absorbant les rayons.

2. Collimateur selon la revendication 1, **caractérisé en ce que** les moyens d'entraînement sont réalisés de sorte qu'un accouplement mécanique forcé garantit l'orientation respective de la partie avant (7, 7') et ainsi des bords avant (5, 5') pour chaque position de la partie arrière (6, 6').

3. Collimateur selon la revendication 1 ou 2, **caractérisé en ce que** les parties avant (7, 7') sont essentiellement des corps demi-circulaires (8, 8') qui sont logés de manière fixe sur l'extrémité avant des parties arrière (6, 6'), le mouvement de réglage étant un basculement autour de l'axe de rotation (36) imaginaire se trouvant au milieu de la ligne de cercle.

4. Collimateur selon la revendication 3, **caractérisé en ce que** la hauteur (10) de la partie arrière (6, 6') correspond sensiblement au diamètre (11) du corps semi-circulaire (8, 8') et les extrémités avant (12) de la partie arrière (6, 6') sont ramenées en position de sorte que chaque position inclinée nécessaire des bords avant (5, 5') des lames d'obturateur (4, 4') est possible.

5. Collimateur selon l'une des revendications 1 à 4, **caractérisé en ce que** les sections transversales des lames d'obturateur (4, 4') présentent une forme trapézoïdale asymétrique (13) telle que ses surfaces latérales (14) s'étendent approximativement parallèlement à la trajectoire de rayon et **en ce que** les surfaces internes (15) des limitations latérales (16) dans la continuité des lames d'obturateur externes s'étendent en oblique de sorte qu'elles se poursuivent dans la continuité sans interruption dans les lames externes d'obturateur (4, 4').

6. Collimateur selon la revendication 5, **caractérisé en ce que** les parties avant (7, 7') présentent respectivement un jeu latéral du fait que leur mouvement de réglage est possible malgré la forme trapézoïdale (13).

7. Collimateur selon l'une des revendications 1 à 6, **caractérisé en ce que** les lames d'obturateur (4, 4') sont coulissantes au-delà de la ligne médiane (17) de l'ouverture possible de collimateur (18).

8. Collimateur selon l'une des revendications 1 à 8, **caractérisé en ce que** chaque lame d'obturateur (4 ou 4') présente un entraînement unique commandable.

9. Collimateur selon l'une des revendications 1 à 7, **caractérisé en ce que** la commande du collimateur (1) en cours de fonctionnement s'effectue par un ordinateur qui adapte le contour et la position de l'ouverture de collimateur (18) à l'objet de rayonnement (20) dans la direction respective de rayonnement, l'ordinateur obtenant les données d'un dispositif pour la saisie de la forme de l'objet de rayonnement (20) et une unité de contrôle vérifiant le résultat du mouvement de réglage.

10. Collimateur selon l'une des revendications 1 á 9, **caractérisé en ce que** les lames d'obturateur (4, 4') sont logées dans un bloc collimateur (19) ou des moitiés de bloc collimateur qui sert (servent) au positionnement de l'ouverture de collimateur (18) par rapport à l'objet de rayonnement (20) et à la source de rayonnement (3).

11. Collimateur selon la revendication 10, **caractérisé en ce que** le bloc collimateur (19) est logé sur un portique (41), un tel mouvement relatif entre le bloc collimateur (19) et le patient (46) étant possible, **en ce que** le patient (46) étant irradiable par adaptation de l'ouverture de collimateur (18) à la forme de l'objet d'irradiation (20) de tous les côtés.

12. Collimateur selon la revendication 2 ou l'une des revendications 3 à 11 avec la revendication 2, **caractérisé en ce que** l'accouplement forcé de l'entraînement des parties arrière (6, 6') des lames d'obturateur (4, 4') s'effectue avec l'entraînement de réglage pour les parties avant (7, 7') par des engrenages.

13. Collimateur selon la revendication 12, **caractérisé en ce que** les engrenages pour les lames d'obturateur (4, 4') au choix sur une lame d'obturateur (4, 4') sont disposés au dessus et dans le cas de la lame d'obturateur adjacente (4, 4') en dessous.

14. Collimateur selon la revendication 12 ou 13, **caractérisé en ce que** l'entraînement de réglage pour les parties avant (7, 7') est réalisé de sorte qu'il dirige ce dernier dans le cas d'un réglage individuel des âmes d'obturateur (4, 4') également en cas de réglage de toutes (4, 4') ou d'une partie des lames d'obturateur (4 ou 4') par rapport à la source de rayonnement (3).

15. Collimateur selon l'une des revendications 12 à 14, **caractérisé en ce que** la partie arrière (6, 6') est associée à une crémaillère d'obturateur (21) dans laquelle s'engage un pignon d'entraînement (23).

16. Collimateur selon la revendication 15, **caractérisé en ce que** la crémaillère (21) associée à la partie arrière (6, 6') est réalisée comme une denture d'un bord longitudinal (37).

17. Collimateur selon la revendication 16, **caractérisé en ce que** dans la zone de la denture des bords longitudinaux (37), la partie arrière attenante (6, 6') est logée décalée en hauteur dans le bloc de collimateur (19) de sorte qu'au-dessus de la denture un élément de guidage (25) relié au bloc de collimateur (19) s'engage dans une rainure de guidage (26) de la partie arrière (6, 6').

18. Collimateur selon l'une des revendications 15 à 17, **caractérisé en ce que** sur la partie avant (7, 7') à l'extérieur de leur axe de rotation (36) est articulée une crémaillère de bord avant (22) dans laquelle s'engage un pignon (23 ou 24), une course de réglage divergente par rapport à la partie arrière (6, 6') étant obtenue, de sorte que l'orientation correspondante du bord avant (5, 5') s'effectue.

19. Collimateur selon la revendication 18, **caractérisé en ce que** la crémaillère d'obturateur (21) et la crémaillère de bord avant (22) sont disposées sur un bord longitudinal (37) de la partie arrière (6, 6') et présentent un pas différent (52, 53, 54) pour obtenir le parcours de réglage différent, un pignon (23 ou 24) s'enclenchant dans les deux crémaillères (21, 22) et la différence de pas étant dans la zone de tolérance de la denture.

20. Collimateur selon la revendication 20, **caractérisé en ce que** pour un engrenage disposé en dessous d'un obturateur (4, 4'), le pas (53) de la crémaillère de bord avant (22) est supérieur au pas (52) de la crémaillère d'obturateur (21).

21. Collimateur selon la revendication 19 ou 20, **caractérisé en ce que** pour un engrenage disposé au dessus d'un obturateur (4, 4'), le pas (53) de la crémaillère de bord avant (22) est inférieur au pas (54) de la crémaillère d'obturateur (21).

22. Collimateur selon l'une des revendications 18 à 21, **caractérisé en ce que** les pignons (23 ou 24) sont logés dans un châssis de base (58).

23. Collimateur selon l'une des revendications 18 à 22, **caractérisé en ce que** les pignons (24) servent simultanément de pignons d'entraînement (23) et sont logés dans le bloc collimateur (19).

24. Collimateur selon la revendication 14 avec l'une des revendications 19 à 23, **caractérisé en ce que** les pignons d'entraînement (23), qui s'engagent dans les crémaillères d'entraînement (55, 21 ou 22), sont logés dans un bloc collimateur mobile (19), ou deux moitiés de bloc collimateurs mobiles et d'autre pignons (24), qui s'engagent dans les crémaillères d'obturateur (21) et dans les crémaillères de bord avant (22), sont logés sur un châssis de base sur lequel le bloc collimateur (19) dans son ensemble ou en en tant que moitiés de bloc collimateur - sont coulissants pour une partie respective des lames d'obturateur (4 ou 4').

25. Collimateur selon l'une des revendications 14 à 17, **caractérisé en ce que** des commandes de coulisse servent au déplacement de réglage des parties avant (7, 7').

26. , Collimateur selon la revendication 25 avec la revendication 15, **caractérisé en ce que** les guides de coulisse (2) sont reliés de manière fixe (61) avec le logement des pignons d'entraînement (23) et les pièces coulissantes (28) de la commande de coulisse sont reliées activement avec la partie avant (7, 7').

27. Collimateur selon la revendication 25 avec la revendication 15, **caractérisé en ce que** les guides de coulisse (27) sont reliés fixement avec un châssis de base (58) et des moities coulissantes de collimateur - pour chaque partie des lames d'obturateurs (4 ou 4') sont reliées fixement avec les logements des pignons d'entraînement (23).

28. Collimateur selon la revendication 26 ou 27, **caractérisé en ce que** la pièce coulissante (28) est fixée sur un treuil (29) qui est guidé en direction de la partie avant (7, 7') et qui est relié par une extrémité (30) au dessus et par l'autre extrémité (31) en dessous de l'axe de rotation imaginaire (36) de la partie avant (7, 7').

29. Collimateur selon la revendication 26 ou 27, **caractérisé en ce que** la pièce coulissante (28) est fixée sur une extrémité arrière (34) d'un levier double (32), le levier (32) étant logé par son axe de rotation (33) sur la partie arrière (6, 6') et par son extrémité avant (35) dans la zone de la partie avant (7, 7').

30. Collimateur selon l'une des revendications 1 à 29, **caractérisé en ce que** sur au moins un bord longitudinal (37) d'une partie arrière (6, 6'), il est prévu un guide (38).

31. Collimateur selon la revendication 30, **caractérisé en ce que** le guide (38) se compose d'une rainure (39) dans le bord longitudinal (37) dans laquelle glisse un élément de guidage (40).

32. Collimateur selon l'une des revendications 1 à 31, **caractérisé en ce que** les lames d'obturateur (4, 4') servent de moyens compensateurs pour la génération d'une intensité de rayonnement différente dans le fait qu'elle sont introduisibles dans l'ouverture de collimateur (18) de temps en temps pendant le rayonnement.
